# EUROPEAN PATENT APPLICATION

(11) **EP 4 527 843 A1**
(43) Date of publication of application: **26.03.2025**
(21) Application number: 23198190.3
(22) Date of filing: 19.09.2023
(51) Int. Cl.: C07F 9/09, C07F 9/10, A61K 9/127

(54) **STEREOISOMERIC PHOSPHATIDYLOLIGOGLYCEROL**

(71) Applicant: Thermosome GmbH, 82152 Planegg (DE)
(72) Inventor: PETROV, Orlin, 82152 Planegg (DE); HOSSANN, Martin, 82152 Planegg (DE)
(74) Representative: Weickmann & Weickmann PartmbB

(57) **Abstract**

The present invention relates to a stereoisomeric phosphatidyloligoglycerol having predetermined configuration at at least three stereogenic centers and, in particular, to a phosphatidyloligoglycerol being a single stereoisomer. The present invention further relates to a stereospecific method for providing a stereospecific phosphatidyloligoglycerol having predetermined configuration at at least three stereogenic centers and, in particular, for providing a phosphatidyloligoglycerol being a single stereoisomer, as well as to liposomes comprising the novel stereoisomeric phosphatidyloligoglycerol and, in particular, to liposomes comprising a novel stereoisomeric phosphatidyloligoglycerol being a single stereoisomer.

## Description

The present invention relates to a stereoisomeric phosphatidyloligoglycerol having predetermined configuration at at least three stereogenic centers and, in particular, to a phosphatidyloligoglycerol being a single stereoisomer. The present invention further relates to a stereospecific method for providing a stereoisomeric phosphatidyloligoglycerol having predetermined configuration at at least three stereogenic centers and, in particular, for providing a phosphatidyloligoglycerol being a single stereoisomer, as well as to liposomes comprising the novel stereoisomeric phosphatidyloligoglycerol and, in particular, to liposomes comprising a novel stereoisomeric phosphatidyloligoglycerol being a single stereoisomer.

Phosphatidyloligoglycerols (PGₓ) being 1,2-diacyl-*sn*-glycero-3-phospho-oligoglycerols, such as 1,2-dipalmitoyl-*sn*-glycero-3-phospho-oligoglycerol (DPPGₓ) are non-natural occurring glycerophospholipids. Phosphatidyloligoglycerols are comprised of a diacylglycerol group, a phosphate group and an oligoglycerol group including two or more glycerol moieties.

Phosphatidyloligoglycerols were in particular developed as constituents of liposomes to increase circulation half-life of liposomes (WO 97/30058 A1). The headgroup of natural occurring phosphatidylmonoglycerols, such as 1,2-dipalmitoyl-*sn*-glycero-3-phosphoglycerol (DPPG) was modified by additional glycerol units connected via ether bonds in order to increase head group hydrophilicity. DPPGₓ can be used to replace PEGylated phospholipids in cholesterol-containing (WO 97/30058 A1) and cholesterol-free liposomes (Hossann et al. 2021) to prolong the circulation half-life of these nanocarriers. An important advantage of phosphatidyloligoglycerols, such as DPPGₓ over PEGylated lipids is the significantly smaller head group (74 Da for each glycerol unit versus approximately 2000 Da for PEG). As result, 1,2-diacyl-*sn*-glycero-3-phospho-oligoglycerols, such as 1,2-dipalmitoyl-*sn*-glycero-3-phospho-diglycerols (DPPG₂), such as 1,2-dipalmitoyl-*sn*-glycero-3-phospho-triglycerols (DPPG₃), such as 1,2-dipalmitoyl-*sn*-glycero-3-phosphotetraglycerols (DPPG₄), form lamellar structures (Lehnert et al. 2003) instead of micellar structures as seen with PEGylated lipids. DPPG₂ could therefore be incorporated into thermosensitive liposome (TSL) formulations up to 70 mol% (Lindner et al. 2004). The impact of DPPG₂ and PEGylated lipids as constituents of liposomes on protein corona, blood cell interactions, complement activation in human plasma/blood and hypersensitivity reactions in rat was recently investigated (Lokerse et al. 2021). Usage of PEGylated liposomes in human drug products was questioned in recent years for several reasons (Hoang et al. 2020), therefore alternatives like DPPGₓ are of interest as novel excipients for liposomal drug delivery systems.

Phosphatidyloligoglycerols, and in particular DPPGₓ (x≥2), can be manufactured by organic synthesis without using enzymes or polymerization reactions (Hossann et al. 2021), thus preventing occurrence of mixed oligoglycerols in a lipid batch. A synthesis route to 1,2-dipalmitoyl-*sn*-glycero-3-phospho-*rac*-oligoglycerols is depicted in WO 97/30058 A1. This route lacked control over the stereocenters in the glycerol unit.

WO 2014/202680 A1 provides a synthesis route with control over two stereogenic centers resulting in 1,2-dipalmitoyl-*sn*-glycero-3-phospho-*sn*-1'-diglycerol. However, the third and further stereogenic centers in the oligoglycerol unit are still racemic due to the lack of stereocontrol in the synthesis of the oligoglycerol building block.

DPPGₓ, and in particular DPPG₂, became of interest in the (pre)clinical development of thermosensitive liposomes (TSLs) with the encapsulated drug doxorubicin (DOX) for the treatment of solid tumors, and in particular soft tissue sarcoma (STS) and bladder cancer (DPPG₂-TSL-DOX) (Hossann et al. 2021, van Valenberg et al. 2021).

WO 2022/008471 A1 describes measures to obtain thermosensitive drug delivery systems based on DPPG₂-TSL with a suitable pharmaceutical quality. A challenge in preclinical development of thermosensitive liposomal formulations for use in human patients is in particular to achieve a stable and long-term storable formulation, without affecting the desired instability at temperatures >39°C for heat-induced localized drug delivery to solid tumors in patients. An optimal formulation has to survive the process of industrial-scale manufacturing and needs to be long-term storable without change in quality-critical specifications that can affect therapeutic efficacy and safety of the patient.

WO 2023/021024 A1 describes an administration schedule for DPPG₂-TSL-DOX in combination with regional hyperthermia (HT) for human patients.

An object of the present invention was to provide novel phosphatidyloligoglycerols having predetermined configuration of all stereocenters in the oligoglycerol headgroup and a method for producing such phosphatidyloligoglycerols in a stereocontrolled manner. Such stereoisomeric phosphatidyloligoglycerols should in particular allow for providing thermosensitive drug delivery systems addressing the requirements and needs identified above.

The present invention relates to a stereoisomeric phosphatidyloligoglycerol of Formula (I) 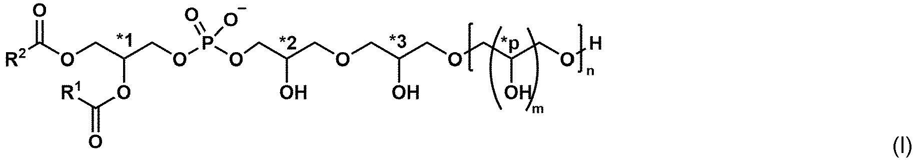
wherein the three stereogenic centers *1, *2 and *3 have a predetermined configuration; and *p denotes further stereogenic centers; and
wherein R¹ and R² each independently are a hydrocarbon group having 12 to 24 C-atoms; n is a number from 0 to 20, in particular, n is a number from 0 to 2; and m at each occurrence independently is 0 or 1.

The inventors of the present application succeeded in providing a stereospecific synthesis for phosphatidyloligoglycerols which allows control over the three stereogenic centers *1, *2 and *3. In case of a diglycerol (n=0), such as a diacylglycerophosphodiglycerol and in particular dipalmitoylglycerophosphodiglycerol, the compound is a single stereoisomer.

The inventive phosphatidyloligoglycerols comprise an oligoglycerol group having at least 2 glycerol moieties. These glycerol moieties include the stereogenic centers *2 and *3. The inventive stereospecific method provided herein allows for the synthesis of a stereoisomeric phosphatidyloligoglycerol of Formula (I) wherein the three stereogenic centers *1, *2 and *3 have a predetermined configuration. The inventive phospholipids may comprise further glycerol and/or glycol groups attached thereto. For m = 1 further glycerol moieties are present, for m = 0 ethylene glycol moieties are included. *p denotes further stereogenic centers in the further glycerol moieties. Preferably each of the further stereogenic centers *p has a predetermined configuration. Thus, most preferred is a phosphatidyloligoglycerol being a single stereoisomer of Formula (I). In such a most preferred phosphatidyloligoglycerol being a single stereoisomer of Formula (I) all stereogenic centers *1, *2, *3 and *p at each occurrence have a predetermined configuration.

According to the invention n is a number from 0 to 20. Preferably, n is a number from 0 to 10, more preferably from 0 to 5. In particular n is 0, 1 or 2, and m = 1, such that the stereoisomeric phosphatidyloligoglycerol of formula (I) is a diglycerol, a triglycerol or a tetraglycerol respectively. Even more preferably n is 0 or 1, and most preferably n is 0. Stereoisomeric phosphatidyloligoglycerols of Formula (I) in which n = 0 are phosphatidyldiglycerols. Stereoisomeric phosphatidyloligoglycerols of Formula (I) in which n = 1 and m = 1 are phosphatidyltriglycerols.

In the stereoisomeric phosphatidyloligoglycerol of Formula (I), residues R¹ and R² each independently are a hydrocarbon group having 12 to 24 C-atoms. Preferably, R¹ and R² each independently are a hydrocarbon group having 13 to 19 C-atoms, in particular 14 to 18 C-atoms. Preferably R¹ and R² each independently are a linear or branched hydrocarbon group, preferably a linear hydrocarbon group. More preferably R¹ and R² each independently are a linear or branched alkyl group, in particular a linear alkyl group. R¹ and R² can be a saturated or a monounsaturated or a polyunsaturated alkyl group, preferably a saturated alkyl group. More preferred, R¹ and R² each independently are a linear saturated alkyl group. Even more preferably, R¹ and R² each independently are a linear saturated C12 to C24 alkyl group, in particular a linear saturated C13 to C19 alkyl group. Most preferably, R¹ and R² are a linear saturated C15 alkyl group.

According to the present invention, the three stereogenic centers *1, *2 and *3 and preferably also the stereogenic center *p have a predetermined configuration. The configuration of each of the three stereogenic centers can be predetermined in either S-configuration or in R-configuration. Most preferred stereogenic center *1 is in R-configuration, stereogenic center *2 is in S-configuration and stereogenic center *3 is in R-configuration. The stereospecific method provided herein allows for the formation of compounds having desired, predetermined configuration at all stereogenic centers. With the inventive method, being stereospecific and not only stereoselective, starting from starting materials being single stereoisomers the product obtained is a phosphatidyloligoglycerol being a single stereoisomer of Formula (I).

In a more general way predetermined configuration in particular means that in a preparation, the respective stereogenic center, e.g. stereogenic center *1 or stereogenic center *2 or stereogenic center *3 is present in the predetermined configuration in a proportion of at least 95 %, preferably of at least 99 %, and most preferably of at least 99.9 %.

The stereoisomeric phosphatidyloligoglycerol of Formula (I) is depicted as anion and can be paired with any cation as counterion. A preferred counterion is Na⁺.

While a desired configuration can be predetermined for each of the three stereogenic centers *1, *2 and *3, most preferred is a stereoisomeric phosphatidyloligoglycerol having Formula (II) 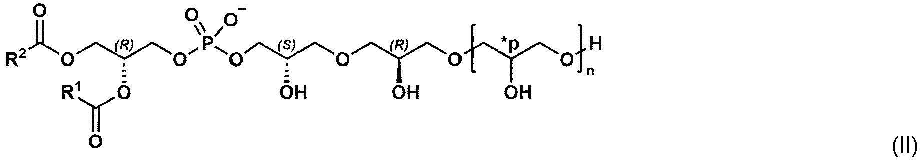 wherein stereogenic center *1 is in R-configuration, stereogenic center *2 is in S-configuration and stereogenic center *3 is in R-configuration and *p denotes further stereogenic centers; and wherein R¹ and R² each independently are a hydrocarbon group having 12 to 24 C-atoms; and wherein n is a number from 0 to 20, in particular is a number from 0 to 2.

Stereoisomeric phosphatidyloligoglycerols having Formula (II) are also named 1,2-diacyl-*sn*-glycero-3-phospho-*sn*-1'-glycero-*sn*-3"-glycerol herein.

The stereospecific method provided herein allows for the formation of 1,2-diacyl-*sn*-glycero-3-phospho-*sn*-1'-glycero-*sn*-3"-glycerol compounds having a desired, predetermined configuration at all stereogenic centers wherein stereogenic center *1 is in R-configuration, stereogenic center *2 is in S-configuration and stereogenic center *3 is in R-configuration. With the inventive method, being stereospecific and not only stereoselective, starting from starting materials being single stereoisomers the product obtained is a phosphatidyloligoglycerol being a single stereoisomer of Formula (II).

In a more general way in the stereoisomeric phosphatidyloligoglycerol having Formula (II) a proportion of at least 95%, preferably of at least 99% and most preferably of at least 99.9% of stereogenic center *1 is in R-configuration, of stereogenic center *2 is in S-configuration and of stereogenic center *3 is in R-configuration.

Most preferred, according to the present invention, is a phosphatidyloligoglycerol being a single stereoisomer of Formula (III)

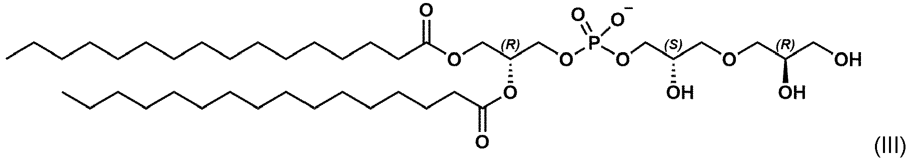

This compound is also termed stereoisomeric DPPG₂ or DPPG₂^{***} or 1,2-dipalmitoyl-*sn-*glycero-3-phospho-*sn*-1'-glycero-*sn*-3"-glycerol herein. The IUPAC name is (2R)-2,3-bis(palmitoyloxy)propyl (2S)-3-{[(2R)-2,3-dihydroxypropyl] oxy}-2-hydroxypropyl phosphate or (2R)-2,3-bis(hexadecanoyloxy)propyl (2S)-3-{[(2R)-2,3-dihydroxypropyl]oxy}-2-hydroxypropyl phosphate. In case of Na⁺ as counterion the IUPAC name is sodium (2R)-2,3-bis(palmitoyloxy)propyl (2S)-3-{[(2R)-2,3-dihydroxypropyl] oxy}-2-hydroxypropyl phosphate or sodium (2R)-2,3-bis(hexadecanoyloxy)propyl (2S)-3-{[(2R)-2,3-dihydroxypropyl] oxy}-2-hydroxypropyl phosphate.

The inventive stereoisomeric phosphatidyloligoglycerols of Formula (I), Formula (II) or Formula (III), respectively, show surprising advantages. In particular, the stereoisomeric phosphatidyloligoglycerols of Formula (I), (II) and (III), show advantages compared to phosphatidyl-*rac*-oligoglycerols.

In particular, the stereoisomeric phosphatidyloligoglycerols show a difference in vitro regarding the temperature-dependent drug release and in vivo regarding pharmacokinetic (PK), organ distribution, and therapeutic efficacy of TSL formulations comprising of such stereoisomeric phosphatidyloligoglycerols and a cytotoxic drug. Moreover, there was a trend for a slower metabolization rate in vitro.

Liposomes containing stereoisomeric phosphatidyloligoglycerols show an altered plasma clearance in vivo. Further, stereoisomeric phosphatidyloligoglycerols show different biological interactions with complement systems and/or plasma proteins compared to racemic molecules. Further liposomes containing stereoisomeric phosphatidyloligoglycerols show a different uptake in organs, in particular in spleen.

Further, it was found that the inventive stereoisomeric phosphatidyloligoglycerols show differences in liposome packing and structural liposome differences. In particular a sharper phase transition in the temperature dependent release profile was observed for liposomes comprising the inventive stereoisomeric phosphatidyloligoglycerols indicating an effect on packing of the lipid excipients.

In particular, the provision of stereoisomeric phosphatidyloligoglycerols of Formula (I) and in particular of Formula (II) and (III) allows for their use in liposomal composition also for human application, even when considering strict criteria as specified by drug administration authorities, by addressing absolute stereochemistry. Chirality of lipid excipient plays a role in the stereoselective metabolism by enzymes (e.g., phospholipases), which is important and prevents a possible accumulation of lipid excipients in patients.

The present invention further relates to a stereospecific method for preparing a stereoisomeric phosphatidyloligoglycerol as described herein and in particular a stereoisomeric phosphatidyloligoglycerol of Formula (I) 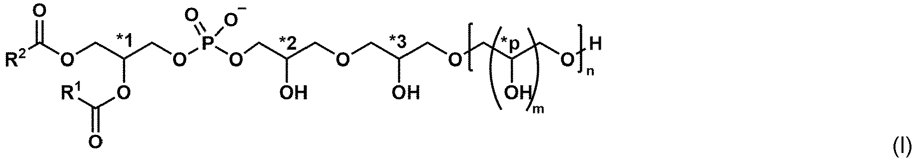
wherein the three stereogenic centers *1, *2 and *3 have a predetermined configuration; and *p denotes further stereogenic centers; and
wherein R¹ and R² each independently are a hydrocarbon group having 12 to 24 C-atoms; n is a number from 0 to 20, in particular, n is a number from 0 to 2; and m at each occurrence independently is 0 or 1;
characterized in that
the synthesis route includes a central intermediate of Formula (IV) or of Formula (IV') 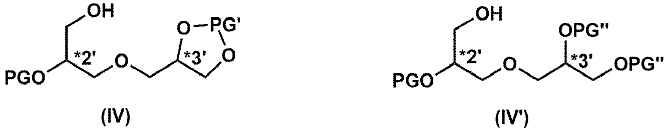
wherein the two stereogenic centers *2' and *3' have a predetermined configuration, and wherein PG, PG' and PG" are independently a protecting group. PG and PG" in particular is benzyl (Bn), tetrahydropyranyl (THP), ethoxyethyl (EE), 2-methoxypropan-2-yl (MOP), silyl or para-methoxybenzyl (PMB), preferably PG and PG" is benzyl (Bn). PG' in particular is CH₂ (methylene), CHCH₃ (ethylidene), CHPh (benzylidene), CHPh*p*-Ome (para-methoxybenzylidene), C(CH₃)₂ (isopropylidene) or Si*t*Bu₂ (di-tert-butylsilylene), Si(iPr)₂-O-Si(iPr)₂ (tetraisopropyldisiloxanylidene), preferably PG' is C(CH₃)₂.

According to the invention, it was found that a synthesis route including a central intermediate of Formula (IV) or (IV'), wherein the two stereogenic centers *2' and *3' have a predetermined configuration allows for stereospecific preparation of phosphatidyloligoglycerols, wherein the three stereogenic centers *1, *2 and *3 can be provided with a predetermined configuration.

The central intermediate of Formula (IV) or (IV') can be prepared from glycidol and 1,2-isopropylidene-glycerol. The configuration of the stereo centers *2' and *3' in the central intermediate of Formula (IV) or (IV') is derived from the configuration of the employed enatiomerically pure starting materials glycidol and 1,2-isopropylidene-glycerol (as described below).

For obtaining a stereoisomeric phosphatidyldiglycerol the central intermediate of Formula (IV) or (IV') is reacted with a compound of formula (V), in particular preferably with compound 12a. For obtaining higher oligoglycerols, in particular tri- or tetraglycerols, the intermediate of Formula (IV) or (IV') is repeatedly reacted with a glycidol to form building blocks having an increased number of glycerol units. In particular, for preparing a stereoisomeric phosphatidyltriglycerol, compound 10a as a building block is provided and for preparing a stereoisomeric phosphatidyltetraglycerol a building block 11a is provided.

A synthesis overview is provided in the schemes below.

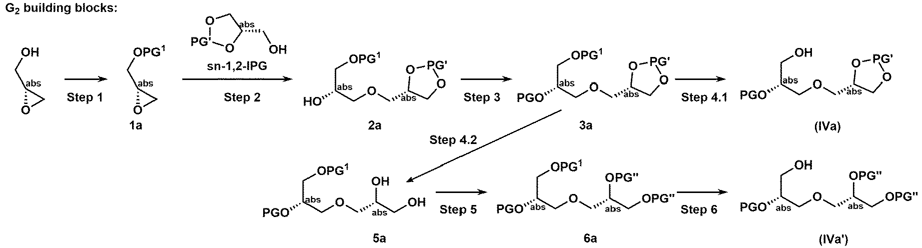

### G₃ building block:

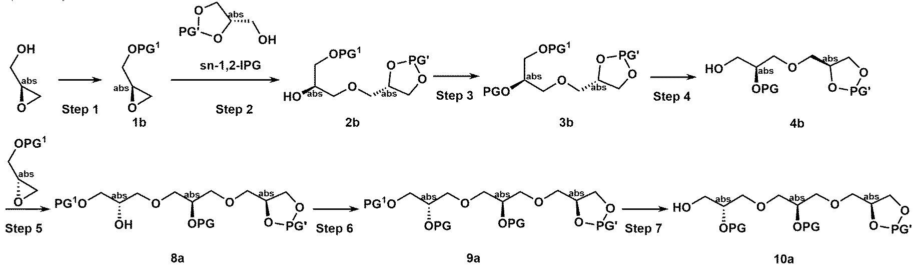

### G₄ building block:

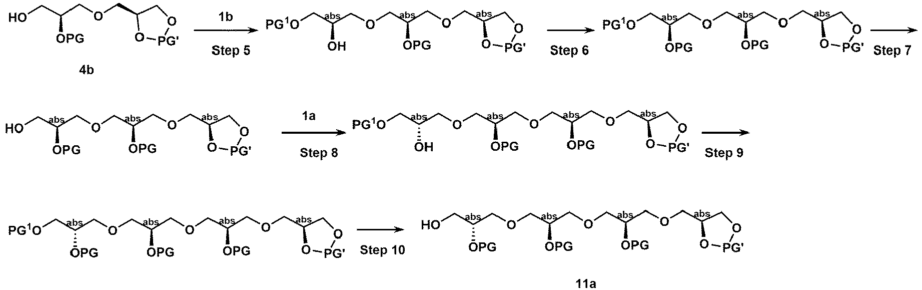

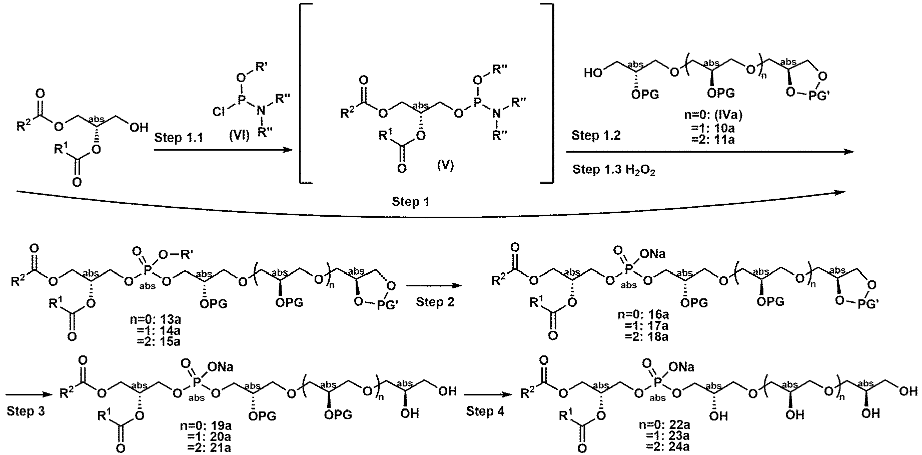

To obtain a stereoisomeric phosphatidyloligoglycerol according to Formula (II) 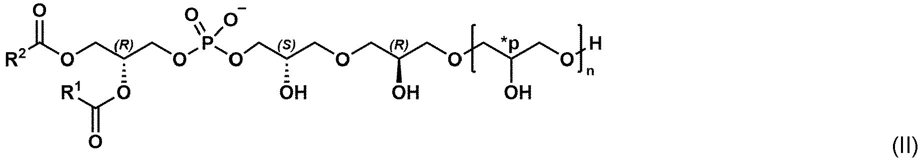 wherein stereogenic center *1 is in R-configuration, stereogenic center *2 is in S-configuration and stereogenic center *3 is in R-configuration, in particular the synthesis route includes a central intermediate of Formula (IVa) or (IVa') 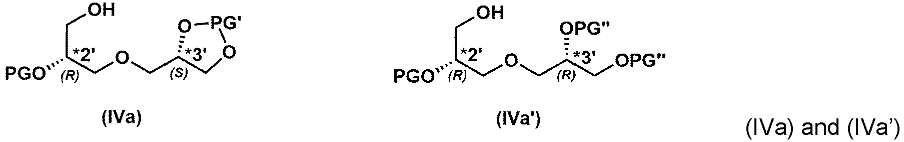 wherein stereogenic center *2' is in R-configuration and stereogenic center *3' is in S-configuration in (lVa) and R-configuration in (IVa'), and wherein PG, PG' and PG" are independently a protecting group.

Preferably, the central intermediate of Formula (IVa) or (IVa') is prepared from glycidol and 1,2-isopropylidene-glycerol, in particular from (R)-(+)-glycidol and (S)-(+)-1,2-isopropylidene-glycerol (*sn*-1,2-IPG) resulting in central intermediate of formula (IVa) or (IVa'), respectively.

Most preferred is central intermediate 4.1a.

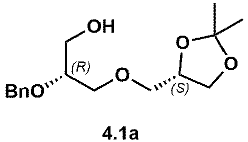

Further, the inventive method particularly comprises reacting a central intermediate of Formula (IV) or (IV'), in particular of Formula (IVa) or (IVa') with a compound of Formula (V) 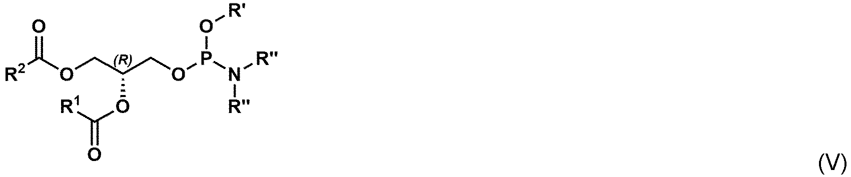 wherein R¹ and R² each independently are a hydrocarbon group having 12 to 24 C-atoms. R¹ and R² are preferably as defined above. R' denotes a leaving group LG. R' in particular is 2-cyanoethyl (CNE). R" is a C1-C12 hydrocarbon group, in particular R" is methyl (Me), ethyl (Et), isopropyl (iPr) or benzyl (Bn), preferably R" is isopropyl.

Preferably, the compound of Formula (V) is prepared from a 1,2-diacyl-sn-glycerol and a phosphorylation reagent of the following structures: 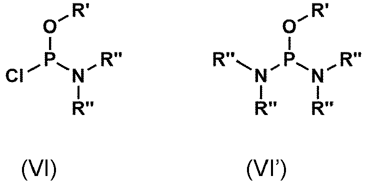 wherein R' and R" independently are as defined above. R' in particular is 2-cyanoethyl (CNE). R" in particular is methyl (Me), ethyl (Et), isopropyl (iPr) and benzyl (Bn), preferably R" is isopropyl.

Most preferred is intermediate 12a, 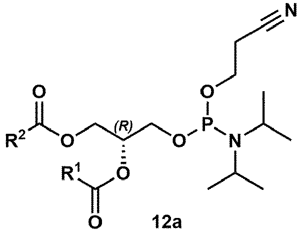 resulting from reaction of 1,2-diacyl-*sn*-glyerol and 2-Cyanoethyl N,N-diisopropylchloro-phosphoramidite (PAMCI).

The inventive method for preparing a stereoisomeric phosphatidyloligoglycerol in particular comprises at least one or more of the process steps as outlined herein.

In particular, the present invention relates to a synthesis for preparing stereoisomeric DPPG₂ having Formula (III)

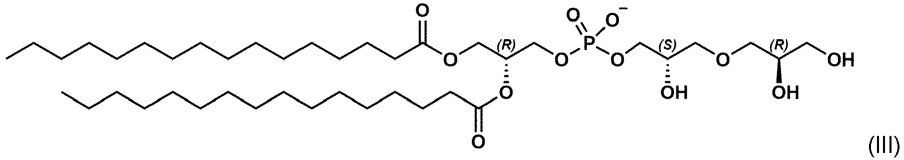

The central intermediate of Formula (lVa) has 2 chiral centers. The configuration of both originates from the enantiomerically pure starting materials, (R)-(+)-glycidol and (S)-(+)-1,2-isopropylidene-glycerol (*sn*-1,2-IPG), and is transferred without isomerization in the final product 1,2-dipalmitoyl-*sn*-glycero-3-phospho-*sn*-1'-glycero-*sn*-3"-glycerol (also termed stereoisomeric DPPG₂ herein). The central intermediate of Formula (IVa) has the (2R),(6S)-configuration and is used in the stereospecific synthesis of stereoisomeric DPPG₂. This single stereoisomer can be obtained in 4 steps with the synthesis shown below. 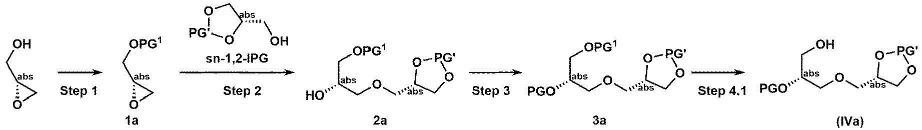 PG, PG¹ and PG' are independently protecting groups.

This route allows control over the 2 stereogenic centers, generating the central intermediate of Formula (IVa) as a single stereoisomer (R)-(+)-glycidol and *sn*-1,2-IPG are used as commercially available starting materials. First, the OH-group in (R)-(+)-glycidol is protected, e.g. with 4-methoxybenzyl (PMB) chloride, resulting in formation of **1a. 1a** reacts with *sn*-1,2-IPG under basic conditions to compound **2a.** Subsequently, the newly generated hydroxyl group is protected, e.g. as a benzyl ether, to give compound **3a.** Finally, PMB ether cleavage using 2,3-dichloro-5,6-dicyano-1,4-benzoquinone gives the central intermediate of Formula (IVa).

In the further synthesis DPPG₂ is prepared as a single stereoisomer from the central intermediate of Formula (IVa) and 1,2-dipalmitoyl-sn-glycerol (*sn*-1,2-DPG) using phosphoramidite chemistry.

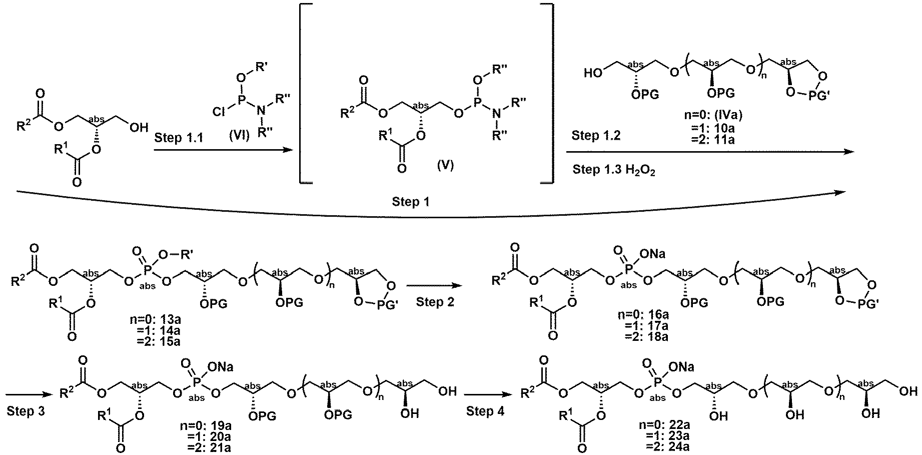

Compound **13a** can be generated in a 3-step one-pot synthesis. For this, preferably first, *sn*-1,2-DPG reacts with 2-Cyanoethyl-*N*,*N*-diisopropylchlorophosphoramidite (PAMCI) to obtain compound **12a.** Central intermediate of Formula (IVa) is added to give the substituted phosphite triester, which is subsequently oxidized, e.g. with HzOz. The so generated fully protected DPPG₂ derivative **13a** is treated to cleave the protecting groups. This can be done according to known procedures. The phosphate protecting group can be cleaved with e.g. 1,8-Diazabicyclo[5.4.0]undec-7-ene (DBU) or TEA; the ketal group with acid e.g. TFA, oxalic and formic acid and the benzyl ether by catalytical hydrogenation with Pd. The deprotected product is purified by chromatography and crystallized from hot ethanol to give DPPG₂ (**22a**) as a single stereoisomer.

The inventive stereoisomeric phosphatidyloligoglycerols are in particular suitable as constituents of liposomes. Thus, the invention further relates to a liposome comprising at least one stereoisomeric phosphatidyloligoglycerol as defined herein.

Liposomes are spherical vesicles formed by a membrane bilayer composed by lipid excipients and are widely used and accepted as drug delivery nanocarriers. The inventive stereoisomeric phosphatidyloligoglycerols form lamellar structures and are therefore particularly suitable as a component for liposomes.

It was found that the inventive stereoisomeric phosphatidyloligoglycerols increase circulation half-life of liposomes. Further the oligoglycerol moiety being a highly hydrated group is capable to alter both electrostatic and hydrophobic interactions with blood components when located on the liposomal surface. Protein binding to liposomes results in a so-called protein corona, whereby the protein corona composition can be steered by the content and kind of inventive stereoisomeric phosphatidyloligoglycerols included in a liposome, such as stereoisomeric DPPG₂. Further liposomes comprising at least one inventive stereoisomeric phosphatidyloligoglycerol show a strong negative surface charge which imparts a high dispersion stability.

The inventive stereoisomeric phosphatidyloligoglycerols can in particular be included in stimuli-sensitive, preferably thermosensitive liposomes. By using stereoisomeric phosphatidyloligoglycerols, it has been possible to prepare thermosensitive liposomes having a prolonged blood circulation time. These liposomes are stable in the bloodstream under physiological conditions (37-38 °C), and do not release, or release only insubstantially, an active ingredient previously enclosed in the liposome. The majority of the liposomes remains in the blood circulation over a period of 2 hours or longer and is therefore available for heat-controlled release of the active ingredient. Owing to the rapid release-kinetics of these liposomes with heat-induced release of the active ingredient within a few seconds, preferably < 30 seconds, more preferably < 20 seconds, a previously enclosed active ingredient can be released immediately by targeted heating of the liposomes to temperatures > 39°C, preferably 40 to 42 °C.

A liposome, in particular a thermosensitive liposome, according to the invention preferably comprises (i) at least one stereoisomeric phosphatidyloligoglycerol as described herein and (ii) at least one additional neutral or zwitterionic phospholipid, preferably a phosphatidylcholine. Preferably the phosphatidylcholine has a main transition temperature of from 0 °C to 80 °C, in particular from 37°C to 45°C.

Thermosensitive liposomes, which comprise (i) at least one stereoisomeric phosphatidyloligoglycerol and (ii) at least one phosphatidylcholine have a long half-life in serum. In addition, the contents of such nanocarrier systems, in particular liposomes, are released rapidly, in particular in less than 30 seconds, under the exertion of a stimulus, for example when a particular temperature is exceeded.

The amount of stereoisomeric phosphatidyloligoglycerol is preferably at least 1 mol%, more preferably at least 10 mol%, yet more preferably at least 15 mol%, and most preferred at least 25 mol% and up to 70 mol%, more preferably up to 50 mol% and yet more preferably up to 35 mol%, based on the lipid content of the liposomes.

A further preferred constituent of liposomes and in particular of thermosensitive liposomes of the invention is at least one phosphatidylcholine. The at least one phosphatidylcholine is present preferably in an amount of at least 1 mol%, more preferably of at least 10 mol%, yet more preferably of at least 30 mol%, even more preferably of at least 50 mol%, in particular at least 60 mol % and most preferably at least 65 mol % and up to 99 mol%, more preferably up to 90 mol% and particularly preferably up to 80 mol%, and most preferably up to 75 mol % based on the lipid content of the liposomes.

Particularly preferred are liposomes comprising 25 to 35 mol % of stereoisomeric phosphatidyloligoglycerols, in particular stereoisomeric DPPG₂, and 65 to 75 mol % phosphatidylcholines based on the lipid content of the liposomes.

The liposome, in particular the thermosensitive liposome, according to the invention preferably comprises at least one phosphatidylcholine of formula (VII) 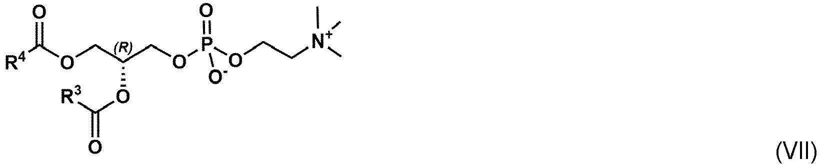 wherein R³ and R⁴ each independently represent a hydrocarbon functional group having from 12 to 24 carbon atoms.

R³ and R⁴ are preferably each independently of the other a saturated or mono- or polyunsaturated, preferably linear alkyl group, in particular a saturated linear alkyl group. R³ and R⁴ are further preferably each independently a C13 to C19, in particular a C15 to C17 hydrocarbon group. Preferably, R³ and R⁴ are each independently a linear saturated C13-to C23-, in particular a C13- to C21-alkyl group. Most preferably, R³ and R⁴ are independently a linear saturated C15- or C17-alkyl group.

The liposome, and in particular the thermosensitive liposome according to the invention, preferably comprises a phosphatidylcholine of formula (VII) in the natural configuration.

Suitable phosphatidylcholines are, for example, 1-palmitoyl-2-oleoyl-*sn*-glycero-3-phosphocholine, 1-stearoyl-2-oleoyl-*sn*-glycero-3-phosphocholine, 1-palmitoyl-2-lauroyl-*sn*-glycero-3-phosphocholine, 1-behenoyl-2-oleoyl-*sn*-glycero-3-phosphocholine, 1-stearoyl-2-lauroyl-*sn*-glycero-3-phosphocholine, 1,3-dimyristoyl-*sn*-glycero-2-phosphocholine, 1,2-dimyristoyl-*sn*-glycero-3-phosphocholine, 1-palmitoyl-2-myristoyl-*sn*-glycero-3-phosphocholine, 1-stearoyl-2-myristoyl-*sn*-glycero-3-phosphocholine, 1-myristoyl-2-palmitoyl-*sn-*glycero-3-phosphocholine, 1,3-palmitoyl-*sn*-glycero-2-phosphocholine, 1,2-dipalmitoyl-*sn-*glycero-3-phosphocholine, 1-myristoyl-2-stearoyl-*sn*-glycero-3-phospho-choline, 1-stearoyl-3-myristoyl-*sn*-glycero-2-phosphocholine, 1-stearoyl-2-palmitoyl-*sn*-glycero-3-phosphocholine, 1-palmitoyl-2-stearoyl-*sn*-glycero-3-phospho-choline, 1,3-distearoyl-*sn-*glycero-2-phosphocholine, 1,2-distearoyl-*sn*-glycero-3-phosphocholine, 1,2-diarachinoyl-*sn*-glycero-3-phosphocholine, 1,2-dibehenoyl-*sn*-glycero-3-phosphocholine and 1,2-dilignoceroyl-*sn*-glycero-3-phosphocholine. 1,2-dipalmitoyl-*sn*-glycero-3-phospho-choline (DPPC) and 1,2-distearoyl-*sn*-glycero-3-phosphocholine (DSPC) are particularly preferred.

The thermosensitive liposome, particularly preferably comprises at least one phosphatidylcholine having a main transition temperature in the range of from 35 °C to 43 °C, yet more preferably in the range of from 39 °C to 41 °C. Thermosensitive liposomes, which comprise such phosphatidylcholines have a release temperature for the contents which allows the liposomes to be stable in normal circulation (at 37 °C) in a healthy human being and to release their contents owing to the action of heat, in particular the local action of heat, at temperatures above 39 °C to 43 °C. Thermosensitive liposomes, having a release temperature of from 40 to 43 °C are particularly preferable.

Thermosensitive liposomes, according to the invention particularly preferably comprise at least one phosphatidylcholine selected from 1,3-dipalmitoyl-*sn*-glycero-2-phosphocholine, and 1,2-dipalmitoyl-*sn*-glycero-3-phospholcholine (DPPC), 1,3-distearoyl-*sn*-glycero-2-phosphocholine and 1,2-distearoyl-*sn*-glycero-3-phosphocholine (DSPC).

Generally various further excipients can be included in the liposomal formulations such as a phosphatidylethanolamine (PE), a phosphatidylglycerol, and a phosphatidylserine (PS); as well as cholesterol (Chol), 1,2-dioleoyl-3-trimethylammonium-propane (DOTAP), 1,2-distearoyl-*sn*-glycero-3-phosphoethanolamine-N-[methoxy(polyethylene glycol)-2000] (DSPE-PEG2000), 1-acyl-*sn*-glycero-3-phosphocholine (Lyso-PC), hexadecyl-phosphocholine (HePC), or polyoxyethylene (20) stearyl ether (Brij78).

Further the liposome may contain cholesterol. However, the most preferred inventive liposome does not contain cholesterol. The liposomes, according to the invention, are preferably free of cholesterol, because cholesterol leads to a spreading-out of the solid to liquid disordered phase transition temperature and thus to a broad thermal transition range.

At high cholesterol concentrations the phase transition temperature is suppressed and non-thermosensitive liposomes are formed. In particular, the liposomes, specifically, thermosensitive liposomes, according to the invention comprise cholesterol in an amount of < 0.1 mol%, more preferably < 0.01 mol%. Particularly preferably the liposomes do not contain any cholesterol but are completely free of cholesterol.

In one embodiment thermosensitive liposomes, having a main phase transition temperature of approximately from 40 °C to 43 °C are formed from mixtures of 1,2-dipalmitoyl-*sn-*glycero-3-phosphocholine, optionally 1,2-distearoyl-*sn*-glycero-phosphocholine and stereoisomeric DPPG₂ and/or stereoisomeric DPPG₃. The 1,2-dipalmitoyl-*sn*-glycero-3-phosphocholine serves as the basic matrix for setting the phase transition temperature at approximately 42 °C, the 1,2-distearoyl-sn-glycero-3-phosphocholine leads to a slight increase in the phase transition temperature, and the stereoisomeric DPPG₂ and/or the stereoisomeric DPPG₃ serves to establish dispersion stability, serum stability, stability in the bloodstream, and facilitates the release rates of the encapsulated compounds.

Particularly preferred according to the invention are liposomes comprising from 20 to 40 mol %, preferably from 25 to 35 mol % of stereoisomeric DPPG₂ or of stereoisomeric DPPG₃, and from 65 to 75 mol % in total of phosphatidylcholines selected from 1,2-dipalmitoyl-*sn*-glycero-3-phosphocholine (DPPC) and/or 1,2-distearoyl-*sn*-glycero-3-phosphocholine (DSPC), based on the lipid content of the liposomes.

Most preferred according to the invention are liposomes comprising from 25 to 35 mol % of stereoisomeric DPPG₂, and from 65 to 75 mol % of 1,2-dipalmitoyl-*sn*-glycero-3-phosphocholine (DPPC), based on the lipid content of the liposomes.

Also, most preferred according to the invention are liposomes comprising from 20 to 40 mol %, preferably from 25 to 35 mol % of stereoisomeric DPPG₂, and from 40 to 60 mol %, preferably from 45 to 55 mol % of 1,2-dipalmitoyl-*sn*-glycero-3-phosphocholine (DPPC) and from 15 to 25 mol % of 1,2-distearoyl-*sn*-glycero-3-phosphocholine (DSPC), based on the lipid content of the liposomes.

As a result of the high stability of liposomes including a stereoisomeric phosphatidyloligoglycerol according to the invention in the systemic circulation with only very low non-specific release of the active ingredient, these liposomes are suitable in a very specific manner for the local therapy of tumors, in particular of soft tissue sarcoma and bladder cancer. After intravenous administration of the liposomes according to the invention, the release of a previously enclosed active ingredient at the desired site, for example in soft tissue, can be induced by targeted heating of the desired site.

An active ingredient enclosed in thermosensitive liposomes can be released by targeted heating of the liposomes or the target tissue respectively, i.e. the part of the body where a tumor is located. The site in question can be heated using a wide variety of methods. For example, in addition to simple flushing with warm water, heating can also be carried out by electromagnetic waves, ultrasound, light or by laser techniques.

The heat treatment can be administered in the form of local hyperthermia, regional hyperthermia, or superficial hyperthermia, preferably as regional deep hyperthermia. The heat treatment can in particular be administered in form of ultrasound induced hyperthermia, microwave frequency induced hyperthermia or hyperthermic perfusion, wherein optionally the ultrasound or microwave frequency induced hyperthermia is administered by external administration. Generally, the choice of hyperthermia treatment will depend on the tumor type to be treated and the specific condition in the patient to be treated. Various options of administering hyperthermia are summarised by Paulides et al. (Paulides et. al, 2020)

For hyperthermia, different types of energy may be used to apply heat, including light, laser light, microwave, radiofrequency, and ultrasound.

In regional or locoregional hyperthermia, moderately large volumes of body parts, such as the thorax or pelvis, including the cancerous region as well as the surrounding healthy tissue may be heated. The remainder of the body is kept as close to normal temperature as possible. Regional hyperthermia may for example be administered as regional deep hyperthermia or regional perfusion hyperthermia.

In a preferred embodiment the heat treatment according to the present invention is administered in form of regional deep or locoregional deep hyperthermia.

Preferably the heat treatment according to the invention is administered in form of electromagnetic-based hyperthermia techniques, for example as radio-wave or microwave frequency induced hyperthermia, ultrasound induced hyperthermia or hyperthermic perfusion.

For use of liposomes for the targeted therapy of localized tumors, the physical properties of the liposomes are of critical importance. Thermosensitive liposomes, loaded with active ingredient should be stable at 36 to 37 °C. By a local increase of the temperature, e.g., in the tumor region, to 42 °C, the active ingredient is released rapidly, in particular in < 60 seconds, preferably < 20 seconds. The lipid constituents of the inventive liposomes pass through a phase transition temperature in the region of about 40 °C to 45°C. Below 40 °C, the phospholipids are arranged lamellar and are in a solid gel phase; above that temperature they are in the liquid disordered phase. In the transition region in a narrow temperature range (e.g., between 40 and 43 °C), the active ingredients are spontaneously and rapidly released.

The main transition temperature for 1,2-dipalmitoyl-sn-glycero-3-phosphocholine is approximately 41 °C. By using the inventive stereoisomeric phosphatidyloligoglycerols, in particular in combination with 1,2-distearoyl-*sn*-glycero-3-phosphocholine it has been possible to provide thermosensitive liposomes, having a prolonged circulation time in the blood.

Thermosensitive liposomes are particularly suitable for the local or regional therapy of cancer. Stereoisomeric phosphatidyloligoglycerols as provided herein allow for clinical use in cancer therapy, since it is ensured that the phospholipids have a well-defined structure, defined in terms of structure, fatty acid composition and configuration. In a preferred embodiment the cancer to be treated is selected from soft tissue sarcoma, preferably undifferentiated pleomorphic sarcoma (UPS), liposarcoma (well differentiated liposarcoma, dedifferentiated liposarcoma, myxoid liposarcoma, pleomorphic liposarcoma), leiomyosarcoma, synovial sarcoma, angiosarcoma, epithelioid sarcoma, malignant peripheral nerve sheath tumor (MPNST), rhabdomyosarcoma (alveolar rhabdomyosarcoma, embryonal rhabdomyosarcoma, pleomorphic rhabdomyosarcoma), solitary fibrous tumor, myxofibrosarcoma, fibrosarcoma, uterine sarcoma (uterine leiomyosarcoma, endometrial stromal sarcoma), desmoplastic small round cell sarcoma (DSRCT), desmoids, kaposi sarcoma or bone sarcoma, preferably Ewing sarcoma, osteosarcoma or chondrosarcoma. In a further embodiment, the cancer is bladder tumor.

With the liposomes according to the invention tumors, in particular solid and/or localized tumors, can be treated. Superficial tumors and metastases in particular can be treated with the liposomes according to the invention because they can be heated in a simple manner. However, other tumors, for example tumors in hollow organs, can also be treated. In this case, the heating required to release the contents of the liposomes can be achieved for example by flushing with warm water.

Most preferred is the treatment of soft tissue sarcoma (STS).

The liposomes according to the invention preferably further comprise an active ingredient, in particular, a cytostatic. Most preferred the inventive liposome comprises doxorubicin (DOX) which is a cytotoxic anthracycline. Further suitable cytostatic anthracyclines presently employed in cancer treatment include epirubicin, idarubicin, daunorubicin and mitoxantrone. In further embodiments of the invention other cytostatics such as mitomycin C, gemcitabine, trabectedin, etc. or platinum derivatives such as cisplatin, carboplatin or oxaliplatin can be included in the liposome.

In a further preferred embodiment a thermosensitive liposome according to the invention, comprises an active pharmaceutical ingredient which is selected from the group consisting of anthracyclines such as doxorubicin, daunorubicin, idarubicin, epirubicin aclarubicin, amrubicin, pirarubicin, valrubicin and zorubicin; anthracenediones such as mitoxantrone and pixantrone; antineoplastic antibiotics such as mitomycin and bleomycin; vinca alkaloids such as vinblastine, vincristine and vinorelbine; alkylating agents such as cyclophosphamide and mechlorethamine hydrochloride: campthothecins such as topotecan, irinotecan (CPT-11), lurtotecan, 9-aminocamptothecin, 9-nitrocamptothecin and 10-hydroxycamptothecin; purine and pyrimidine derivatives such as 5-fluorouracil,gemcitabine (2,2'-difluoro-2-deoxycytidine, dFdC), floxuridine (FUDR), cytarabine (cytosine arabinoside), 6-azauracil (6-AU); oxazaphosphorines such as cyclophosphamide, ifosfamide and trofosfamide; taxanes such as paclitaxel and docetaxel; podophyllotoxin derivatives such as etopside and teniposide; platinum-based compounds such as cisplatin, carboplatin, oxaliplatin, nedaplatin; methotrexate; tyrosine kinase inhibitors such as imatinib, gefitinib, erlotinib, sunitinib, adavosertib and lapatinib; and cytarabines such as cytosine arabinoside.

By using liposomes, in particular thermosensitive liposomes, according to the invention, an active ingredient enclosed in the liposomes, can be released by exerting a stimulus, in particular local heating of the liposomes. The liposomes, in particular thermosensitive liposomes, are therefore particularly suitable for the local or regional therapy of tumors, in particular of soft tissue sarcoma. The release of the active ingredient enclosed in the liposomes, can be induced in a targeted manner, so that the active ingredient, in particular a cytostatic, can be released directly at the desired site. These experiences can also be used for the therapy of other solid and localized tumors.

The liposomes described herein are particularly suitable for the local release of active pharmaceutical ingredients by hyperthermia. Liposomes, that are thermosensitive and comprise at least one phosphatidylcholine and at least one stereoisomeric phosphatidyloligoglycerol having a phase transition temperature of about 40 to 43 °C are preferable.

The fundamental requirements for a clinical application are thus met, in particular for use in the regional therapy of tumor diseases, for example soft tissue sarcoma. The effectiveness of these therapies has already been confirmed in animal experiments.

If the phase transition temperature is passed through, the membrane changes phase state and becomes permeable and the liposome contents are accordingly released. This effect can be used according to the invention for treating soft tissue sarcoma. The temperature in the tumor is increased to the temperature needed for release of the liposome contents and the liposome contents are then released specifically and almost exclusively in the tumor, so that the active ingredients can be used effectively for treating the tumor.

The invention therefore further relates to a thermosensitive liposome as described herein in combination with hyperthermia and/or ultrasound. Heating can be applied in this case by a large number of methods, such as simply flushing with warm water, heating by means of electromagnetic waves, ultrasound, light or laser. Preferred are ultrasound induced hyperthermia, microwave frequency induced hyperthermia or hyperthermic perfusion.

The liposomes, in particular thermosensitive liposomes, according to the invention including those liposomes comprising an active pharmaceutical ingredient, may be prepared via a wide range of different techniques such as lipid film hydration, ethanol injection or an extrusion method. The inventive liposomes are preferably prepared by ethanol injection. Further preferred buffer exchange is performed with tangential flow filtration (TFF).

The invention therefore also includes a liposome, in particular thermosensitive liposome, for use in the treatment of other tumors, in particular soft tissue sarcoma.

The cancer is preferably a soft tissue sarcoma (STS). STS are a group of cancers that originates in the tissues that connect, support and surround other body structures, such as muscle, fat, blood vessels, nerves, tendons and the lining of joints. Preferably, the STS may be undifferentiated pleomorphic sarcoma (UPS), liposarcoma, in particular differentiated liposarcoma, dedifferentiated liposarcoma, myxoid liposarcoma, pleomorphic liposarcoma; leiomyosarcoma, synovial sarcoma, angiosarcoma, epithelioid sarcoma, malignant peripheral nerve sheath tumor (MPNST), rhabdomyosarcoma, in particular alveolar rhabdomyosarcoma, embryonal rhabdomyosarcoma, pleomorphic rhabdomyosarcoma; solitary fibrous tumor, myxofibrosarcoma, fibrosarcoma, uterine sarcoma, in particular uterine leiomyosarcoma, endometrial stromal sarcoma; desmoplastic small round cell sarcoma (DSRCT), desmoids, Kaposi sarcoma or bone sarcoma, in particular Ewing sarcoma, osteosarcoma or chondrosarcoma.

The invention further includes a liposome, in particular thermosensitive liposome, the for use in the treatment of other tumors such as osteosarcoma, bladder carcinoma (muscle invasive bladder cancer [MIBC] and non-muscle invasive bladder cancer [NMIBC]), ovarian carcinoma, stomach carcinoma, breast carcinoma (especially triple negative breast cancer [TNBC]), hepatocellular carcinoma, uterine carcinoma, carcinoma of the thyroid gland, head-neck tumors, prostate carcinoma, chordoma, desmoid tumor, glioblastoma and other tumor diseases having preferably locoregional spread.

Such liposomes preferably comprise an active pharmaceutical ingredient suitable for treating the tumor in question, which is then released stimuli-sensitively in or in the vicinity of the tumor.

### 1 References

Brooks WH, Guida WC, Daniel KG. The significance of chirality in drug design and development. Curr Top Med Chem 2011;11:760-70. DOI: 10.2174/156802611795165098
Hoang Thi TT, Pilkington EH, Nguyen DH, Lee JS, Park KD, Truong NP. The Importance of Poly(ethylene glycol) Alternatives for Overcoming PEG Immunogenicity in Drug Delivery and Bioconjugation. Polymers 2020; 12:298. DOI: 10.3390/polym 12020298
Hossann M, Wang T, Wiggenhorn M, Schmidt R, Zengerle A, Winter G, et al. Size of thermosensitive liposomes influences content release. J Control Release 2010; 147:436-43. DOI: 10.1016/j.jconrel.2010.08.013
Hossann M, Hirschberger J, Schmidt R, Baumgartner C, Zimmermann K, Baer S, et al. A heat-activated drug delivery platform based on phosphatidyl-(oligo)-glycerol nanocarrier for effective cancer treatment. Adv NanoBiomed Res 2021;1:2000089. DOI: 10.1002/anbr.202000089
Kneidl B, Peller M, Winter G, Lindner LH, Hossann M. Thermosensitive liposomal drug delivery systems: state of the art review. Int J Nanomedicine 2014;9:4387-98. DOI: 10.2147/IJN.S49297
Lehnert R, Eibl H, Müller K. FT-IR and NMR studies on the conformational and structural properties of 1,2-dipalmitoyl-sn-glycero-3-phosphatidyloligoglycerols. J Phys Chem 2003;107:75-85. DOI: 10.1021/jp026509t
Limmer S, Hahn J, Schmidt R, Wachholz K, Zengerle A, Lechner K, et al. Gemcitabine treatment of rat soft tissue sarcoma with phosphatidyldiglycerol-based thermosensitive liposomes. Pharm Res 2014;31:2276-86. DOI: 10.1007/s11095-014-1322-6
Lindner LH, Eichhorn ME, Eibl H, Teichert N, Schmitt-Sody M, Issels RD, et al. Novel temperature-sensitive liposomes with prolonged circulation time. Clin Cancer Res 2004;10:2168-78. DOI: 10.1158/1078-0432.CCR-03-0035
Lokerse WM, Lazarian A, Kleinhempel A, Petrini M, Schwarz P, Hossann M, et al. Mechanistic investigation of thermosensitive liposome immunogenicity and understanding the drivers for circulation half-life: A polyethylene glycol versus 1,2-dipalmitoyl-sn-glycero-3-phosphodiglycerol study. J Control Release 2021;333:1-15. DOI: 10.1016/j.jconrel.2021.03.014
Nutt DJ, Feetam CL. What one hand giveth the other taketh away: some unpredicted effects of enantiomers in psychopharmacology. J Psychopharmacology 2010;24:1137-41. DOI: 10.1177/0269881110374782
Paulides MM, Dobsicek Trefna H, Curto S, Rodrigues DB. Recent technological advancements in radiofrequency- and microwave-mediated hyperthermia for enhancing drug delivery. Adv Drug Deliv Rev. 2020;163-164:3-18. DOI: 10.1016/j.addr.2020.03.004
Schagon O. Liposomen als potentielle Arzneistofftrager: Variation der biopharmazeutischen Eigenschaften durch 1,2-Dipalmitoyl-sn-glycero-oligo-glycerine. Shaker Verlag, Aachen; 1997. ISBN: 978-3-8265-2479-0
van Valenberg FJP, Brummelhuis ISG, Lindner LH, Kuhnle F, Wedmann B, Schweizer P, et al. DPPG2-based thermosensitive liposomes with encapsulated doxorubicin combined with hyperthermia lead to higher doxorubicin concentrations in the bladder compared to conventional application in pigs: a rationale for the treatment of muscle-invasive bladder cancer. Int J Nanomedicine 2021;16:75-88. DOI: 10.2147/IJN.S280034.
Willerding L, Limmer S, Hossann M, Zengerle A, Wachholz K, Hagen TL ten, et al. Method of hyperthermia and tumor size influence effectiveness of doxorubicin release from thermosensitive liposomes in experimental tumors. J Control Release 2016;222:47-55. DOI: 10.1016/j.jconrel.2015.12.004

The invention is further illustrated by the enclosed figures and examples.

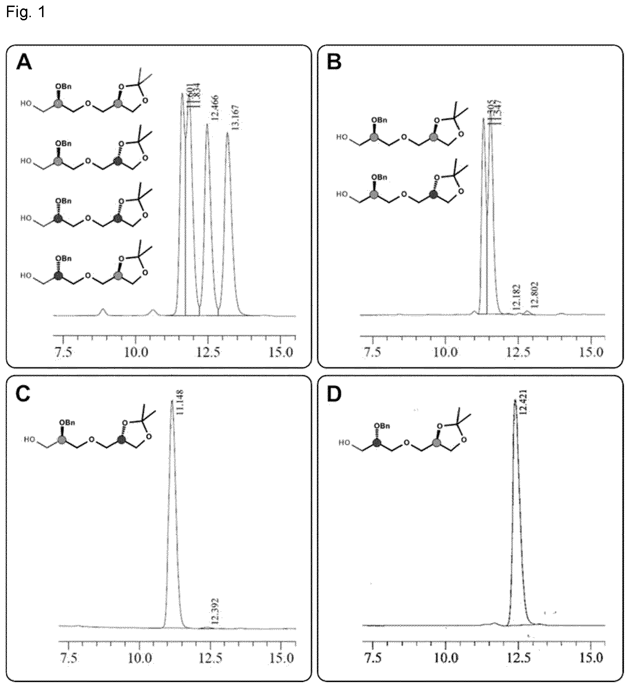

Figure 1: Evaluation of optical purity of central intermediate of Formula (IVa) with chiral HPLC. Fig. 1A shows fully racemic intermediate, Fig. 1B shows a mixture of epimer 2(S),6(R,S) (98.9%), Fig. 1C shows the desired stereoisomer 2(S),6(R) (99.5%), and Fig. 1D shows stereoisomer 2(R),6(S) (100.0%).

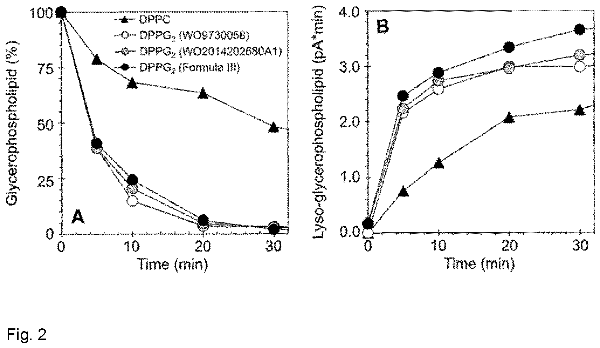

Figure 2: Cleavage of DPPG₂ with phospholipases PLA₂. Fig. 2A shows cleavage of the glycerophospholipid, and Fig. 2B shows formation of the lyso-glycerophospholipid. Values with unit (%) are normalized to the content of glycerophospholipid (% m/m) in time point t=0 min (without phospholipase). In this way the difference in purity and other matters, such as residual solvent or residual matter, is considered.

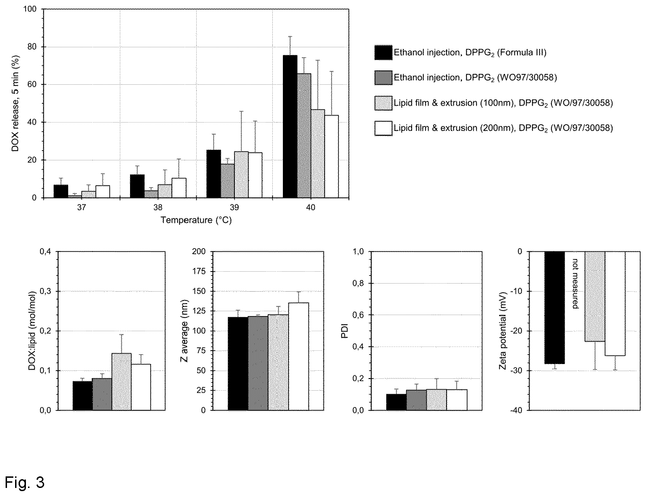

Figure 3: Comparison of biophysical characteristics for distinct DPPG₂-TSL-DOX batches. Batches were produced either by 1) ethanol injection and using DPPG₂ (formula III), 2) ethanol injection and using DPPG₂ (WO97/30058), 3) lipid film hydration with 100 nm extrusion using DPPG₂ (WO97/30058), and 4) lipid film hydration with 200 nm extrusion using DPPG₂ (WO97/30058).

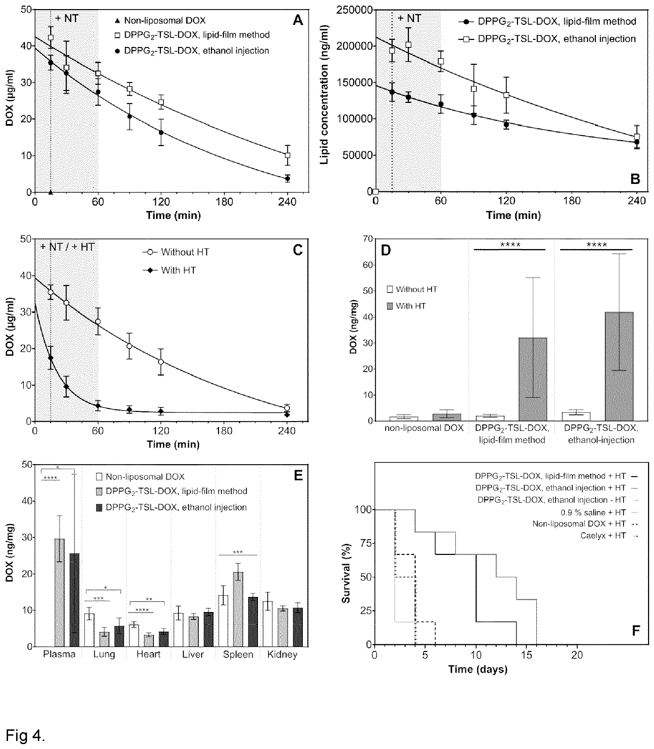

Figure 4: DPPG₂-TSL-DOX in a rat sarcoma model. PK profile of (A) DOX and (B) DPPG₂ after i.v. bolus injection of distinct DOX formulations (2 mg/kg DOX, applied as fixed volume of 1 mL). Application of normothermia (NT) treatment via water bath set at 37 °C for 60 minutes (blue area). N=6 per formulation. (C) PK profile of DOX after application of DPPG₂-TSL-DOX prepared with DPPG₂ according to formula III with the ethanol injection preparation method as i.v. bolus injection of 2 mg/kg DOX. Rats received a 60 min water bath treatment of one hind leg at either 37°C (without HT) or 41.5°C (with HT), respectively. N=6 per formulation. (D) Accumulation of DOX in tumors and (E) organs 60 min after i.v. bolus injection of distinct DOX formulations (2 mg/kg DOX, applied as fixed volume of 1 mL). All animals received lamp HT application for one tumor while the contralateral tumor (other hind leg) received no treatment. N=6 per formulation. Student t-test for statistical analysis with * p<0.05; ** p<0.01; *** p<0.001; **** p<0.0001. (F) Therapeutic efficacy in the BN175 rat sarcoma model of distinct DOX formulations (2 mg/kg DOX) in combination with 60 min lamp HT (41°C) of the tumor. Kaplan-Meier plot (survival, 5-fold increase in tumor volume). NT: Animals received no HT treatment. N=6 per formulation.

### Examples

### 1.1 Prior Art

### 1.1.1 Synthesis of 1,2-Dipalmitoyl-sn-glycero-3-phospho-rac-diglycerol (comparison example)

A synthesis route for manufacturing of DPPG₂ was described in patent application WO 97/30058 A1 as part of the synthesis of glycerophospholipids of the molecule class of phosphatidyloligoglycerols (PGₓ). DPPG₂ was synthesized in a multi-step organic synthesis lacking control over the stereocenters in the glycerol unit.

### 1.1.2 Synthesis of 1,2-Dipalmitoyl-sn-glycero-3-phospho-sn-1'-diglycerol (comparison example)

WO 2014/202680 A1 describes a synthesis route that allowed synthesis of 1,2-dipalmitoyl-*sn*-glycero-3-phospho-*sn*-1'-glycero-3'-rac-glycerol. Objective was to obtain DPPG₂ with a *sn*-1' configuration as in natural occurring PG glycerophospholipids. This configuration is relevant for phospholipase activity and would prevent a possible accumulation of the lipid after application to patients. The synthesis route allows for control over only two of the stereogenic centers in the molecule.

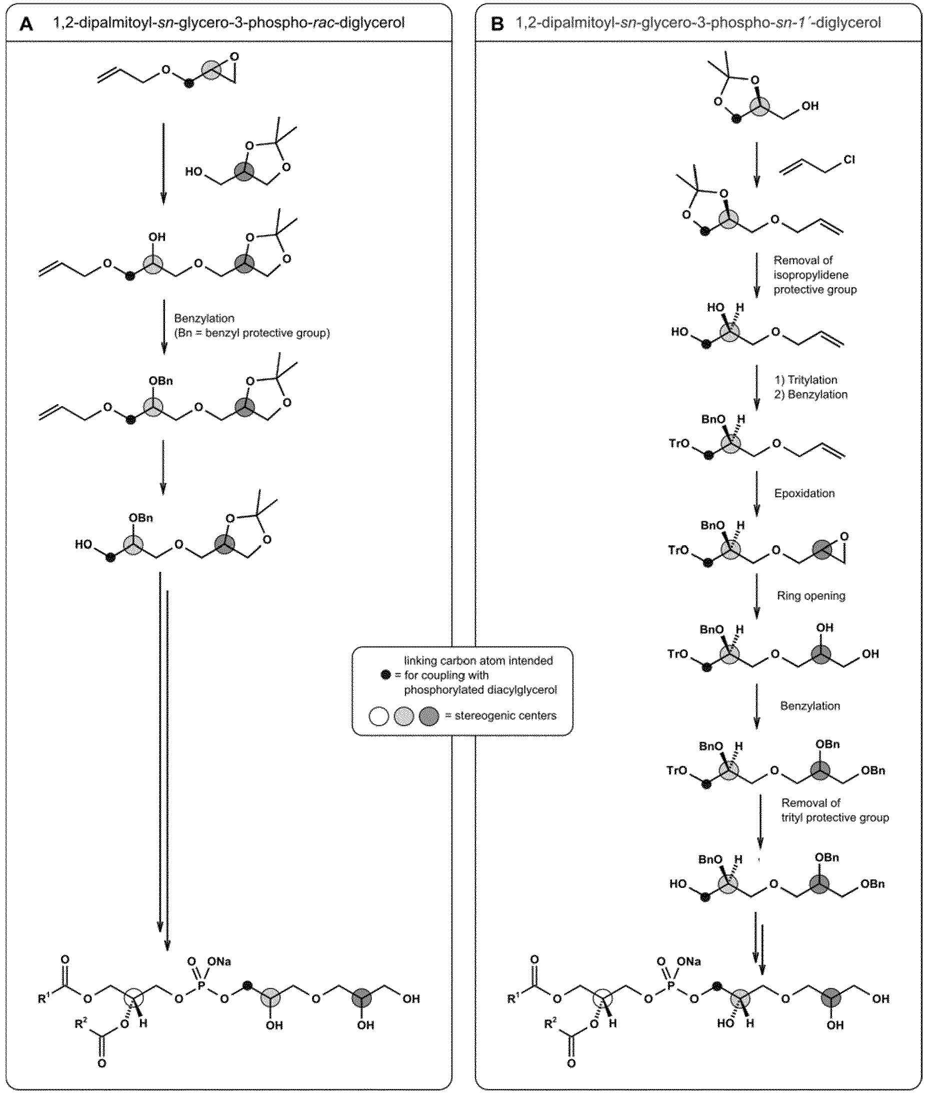

*Comparison of the synthesis route for DPPG₂ disclosed in (A)* WO9730058 (1,2*-dipalmitoyl-sn-glycero-3-phospho-rac-diglycerol) and (8)* WO 2014/202680 A1 *(1,2 dipalmitoyl-sn-glycero-3-phospho-sn-1'-diglycerol). Both routes do not allow control over all three stereogenic centers due to an epoxide ring opening step.*

### 1.2 Synthesis of (2R)-2,3-bis(palmitoyloxy)propyl (2S) 3-{[(2R)-2,3-dihydroxypropyl]oxy}-2-hydroxypropyl) phosphate

### 1.2.1 Manufacturing of the preferred example of central intermediate of formula 4.1a

Intermediate **4.1a,** the preferred version of central intermediate (IVa), has 2 chiral centers with a (2R),(6S)-configuration. The configuration of both chiral centers originates from the enantiomerically pure starting materials (R)-(+)-glycidol and (S)-(+)-1,2-isopropylidene-glycerol (sn-1,2-IPG) and is transferred without isomerization in the final product DPPG₂. The single stereoisomer **4.1a** is obtained in 4 steps with the following synthesis:

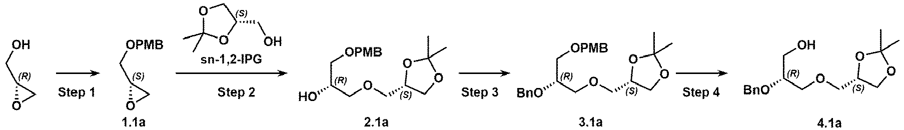

By the following combination of enantiomerically pure starting materials, the other possible stereoisomers are obtained:
**4.1b**: (2S),(6R)-configuration: (S)-(-)-glycidol and (R)-(-)-1 ,2-isopropylidene-glycerol (*sn-*2,3-IPG)
**4.1c**: (2R),(6R)-configuration: (R)-(+)-glycidol and (R)-(-)-1,2-isopropylidene-glycerol (*sn-*2,3-IPG)
**4.1d**: (2S),(6S)-configuration: (S)-(-)-glycidol and (S)-(+)-1,2-isopropylidene-glycerol (*sn-*1,2-IPG)

### Step 1:

3.8 kg (R)-(+)-glycidol are dissolved in 22.8 L NMP, then 8.02 kg PMBCI are added followed by 2.26 kg NaH at 0-5 °C. After 4 hours at RT, the reaction mixture is poured into ice water and extracted with EtOAc. The organic phase is washed with brine, dried over Na₂SO₄ and concentrated under reduced pressure. The isolated residue is purified by silica gel chromatography (n-Heptane/Ethyl acetate) and 8.0 kg of **1.1a** are obtained as colorless oil. It is characterized by HPLC, TLC and ¹H-NMR.

¹H NMR of **1.1a** (400 MHz, CDCl₃):
δ ppm 7.29 (d, *J* = 10.8 Hz, 2H), 6.89 (d, *J* = 10.8 Hz, 2H), 4.53 (q, *J* = 11.6 Hz, 2H), 3.79 (s, 3H), 3.74 (dd, *J* = 3.2, 11.6 Hz, 1H), 3.41 (dd, *J* = 6.0, 11.6 Hz, 1H), 3.17 (m, 1H), 2.79 (dd, *J* = 4.0, 9.2 Hz, 1H), 2.61 (dd, *J* = 2.4, 4.8 Hz, 1H).

### Step 2+3:

8.0 kg of **1.1a** are dissolved in 64 L DMF, then 8.16 kg sn-1,2-IPG is added, followed by 6.94 kg tBuOK at 0-5 °C and the resulting reaction mixture stirred for 12 h at RT to obtain **2.1a.** Characterization is done with TLC and HPLC. Subsequently, without work up or further purification additional 6.91 kg tBuOK and 12.6 kg benzyl bromide are added at 0-5 °C and the resulting reaction mixture stirred at 4 h at RT. Then, the reaction mixture is poured into ice water and extracted with EtOAc. The organic phase is washed with brine, dried over Na₂SO₄ and concentrated under reduced pressure. 7.0 kg of pure compound **3.1a** is obtained as yellow oil after purification by silica gel chromatography (n-Heptane/Ethyl acetate). **3.1a** is characterized with TLC, HPLC, and ¹H-NMR.

¹H NMR of **3.1a** (400 MHz, CDCl₃):
δ ppm 7.39 - 7.26 (m, 8H), 6.92 (d, *J* = 8.4 Hz, 2H), 4.71 (s, 2H), 4.50 (s, 2H), 4.27 (d, *J* = 6.0 Hz, 1H), 4.10 - 4.02 (m, 1H), 3.85 (s, 3H), 3.83 - 3.47 (m, 9H), 1.44 (s, 3H), 1.39 (s, 3H).

### Step 4:

6.9 kg **3.1a** are dissolved in 41.8 L DCM/H₂O 10:1 (vol/vol) and the mixture cooled to 0 °C. 4.89 kg 2,3-dichloro-5,6-dicyano-1,4-benzoquinone (DDQ) is added and the mixture stirred at RT for 4 h. The reaction mixture is filtered and the filtrate washed with aq. NaHCO₃ and water. The organic phase is dried over Na₂SO₄ and the solvent removed under reduced pressure. After work up, crude **4.1a** is purified with silica gel chromatography (n-Heptane/Ethyl acetate) and 2.6 kg pure **4.1a** obtained as brown oil. **4.1a** is characterized by HPLC, TLC and SFC, ¹HNMR.
¹H NMR of **4.1a** (400 MHz, CD₃OD)
δ ppm 7.41 - 7.23 (m, 5H), 4.67 (s, 2H), 4.29 - 4.19 (m, 1H), 4.03 (dd, *J* = 6.4, 8.4 Hz, 1H), 3.76 - 3.69 (m, 1H), 3.68 - 3.56 (m, 5H), 3.55 - 3.46 (m, 2H), 1.37 (s, 3H), 1.32 (s, 3H).

### 1.2.2 Evaluation of the optical purity of the central intermediate

Optical purity of the central intermediate of formula (IVa), in particular 4.1a was proven by chiral HPLC (Hexane/Isopropanol with isopropylamine) using a Chiralpak IA-3 column (3 µm, 0.46 cm ID x 25 cm length) from Daicel Chiral Technologies. Samples of all possible stereoisomers and mixtures thereof have been prepared to allow correct assignment of the corresponding peaks (see figure 1).

### 1.2.3 Manufacturing of DPPG₂ from central intermediate of formula (IVa) and 1,2-dipalmitoyl-sn-glycerol (sn-1,2-DPG)

### Synthesis of DPPG₂ as single stereoisomer:

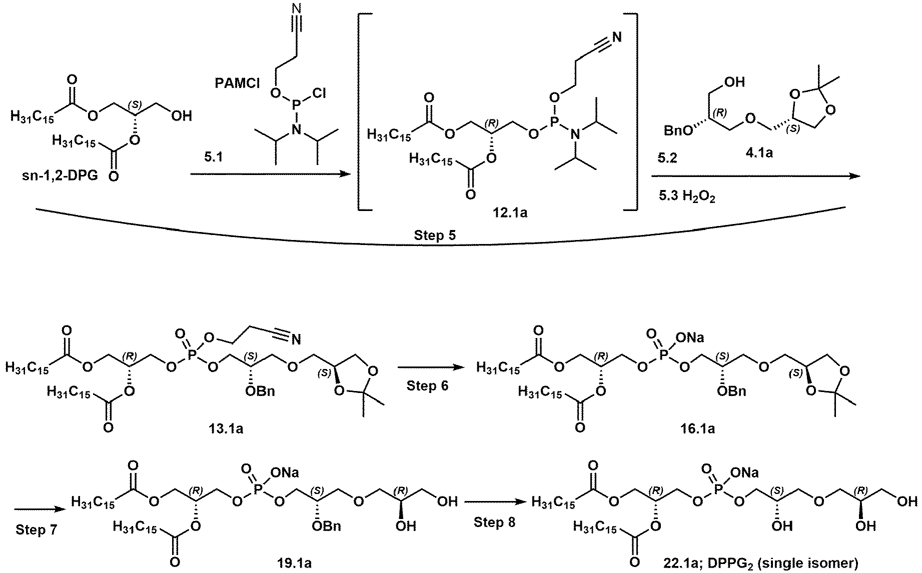

The synthesis allows for control over all 3 stereogenic centers in DPPG₂. Starting materials for introduction of stereogenic centers are 1,2-dipalmitoyl-*sn*-glycerol (*sn*-1,2-DPG) and **4.1a.** The 3 stereogenic centers in DPPG₂ are denoted by (S) and (R) to show absolute stereochemistry.

### Step 5:

230 mL DIPEA are added to a suspension of 500 g *sn*-1,2-DPG in 1 L MeTHF and 1.25 L DCM, then then mixture is cooled to -15 °C. 206 mL 2-Cyanoethyl-*N*,*N-*diisopropylchlorophosphoramidite (PAMCI) are dissolved in DCM and the solution cooled to -20 °C, before addition to the reaction mixture. The resulting mixture is stirred for 1 h at - 15 °C, followed by 1.5 h at 0°C and 1 h at 20 °C. Then 114 g of 4,5-dicyanoimidazole (DCI) and 274 g of **4.1a** are dissolved in 1.5 L MeTHF and the solution slowly added at 20 °C to the reaction mixture. An additional 52 g of DCI is added as solution in 500 ml DCM after stirring for 1 h, then the whole mixture is stirred overnight at 20 °C. Then 91 ml of aqueous 35% w/w H₂O₂ solution are added at 0 °C over 1 h and then stirred at 20 °C for another 1 h. The reaction mixture is diluted with 5 wt% aq. Na₂S₂O₅ solution and extracted with MeTHF. The organic phase is dried over Na₂SO₄ and the solvents are removed under reduced pressure. The crude intermediate **13.1a** is purified by flash silica chromatography (DCM/acetone), resulting 664 g of **13.1a** as white glassy solids.

¹H NMR of **13.1a** (400 MHz, CDCl₃):
δ ppm 7.91 - 7.94 (m,7 H), 7.66 - 7.74 (m, 7 H), 5.31 - 5.40 (m, 15 H), 5.19 - 5.28 (m, 1 H), 2.23 - 2.38 (m, 4 H).

### Step 6:

165 g DBU is added to a solution of 664 g of **13.1a** dissolved in MeTHF and the reaction mixture stirred at RT for 2 h. Then 72 g Pd/C are added, and the obtained reaction suspension is stirred under H₂ atmosphere (1.2 bar) for 1 h. The reaction mixture is filtered over celite and washed with 1M aq. citric acid solution and organic phase separated. The organic phase is washed with 5 wt%. aq. NaHCO₃ solution, dried over Na₂SO₄ and concentrated under reduced pressure. 725 g of crude **16.1a** are obtained.

¹H NMR of **16.1a** (400 MHz, CDCl₃):
δ ppm 7.83 - 8.01 (m, 6 H), 7.63 - 7.79 (m, 6 H), 5.28 - 5.38 (m, 12 H), 5.16 - 5.26 (m, 1 H), 2.19 - 2.33 (m, 4 H).

### Step 7:

707 g of crude **16.1a** are suspended in 1.7 L 2-propanol, then 688 g TFA added, and the obtained reaction mixture stirred at 45 °C for 1 h. The TFA is removed under reduced pressure and the crude product twice co-distilled with 2-propanol, then dissolved in MeTHF and the pH adjusted to 7 with 8 wt% NaHCO₃ solution. The organic phase is separated, dried over Na₂SO₄ and concentrated under reduced pressure to obtain 840 g of crude **19.1a.**
¹H NMR of **19.1a** (400 MHz, CDCl₃)
δ ppm 7.87 - 7.98 (m, 5 H), 7.65 - 7.76 (m, 5 H), 5.28 - 5.40 (m, 11 H), 5.16 - 5.27 (m, 1 H), 4.51 - 4.69 (m, 2 H), 2.14 - 2.32 (m, 4 H), 0.81 - 0.96 (m, 7 H).

### Step 8:

615 g of **19.1a** is dissolved in 2.8 L MeTHF and then washed with 3.1 I 8 wt% NaHSO₄ solution, followed adjustment of the pH to 4 with 60 g NaOAc in 3.1 L water. The organic phase is separated and concentrated to 9.3 L. A suspension of 20% Pd(OH)₂ on charcoal (50% wet; 31.7 g) in 1 L MeOH is added and the obtained suspension is stirred under H₂ atmosphere (3.5 bar) for 18 h. The pH is adjusted to pH 7 with addition of 35 g NaOAc, then the mixture is filtered over decalite and concentrated under reduced pressure. The concentrate is diluted with DCM and filtered over decalite again and then the organic phase is separated and concentrated under reduced pressure. Crude concentrate containing 8 is purified by flash silica chromatography (DCM/MeOH), Pd scavenger treatment and subsequent crystallization from ethanol, which results 322 g of **DPPG₂** as white powder.

¹H NMR of **DPPG₂** (400 MHz, CDCl₃):
δ ppm 7.88 - 7.98 (m, 5 H), 7.66 - 7.74 (m, 5 H), 5.34 (s, 10 H), 5.19 - 5.28 (m, 1 H), 2.21 - 2.37 (m, 4 H).

IPC is performed by HPLC/CAD measurements in every synthesis step. Purification of intermediate **13.1a** by chromatography is implemented to control and adjust the quality of this intermediate if necessary.

### 1.3 Synthesis of (R)-2,3-bis(palmitoyloxy)propyl ((S)-3-((R)-3-((R)-2,3-dihydroxypropoxy)-2-hydroxypropoxy)-2-hydroxypropyl) phosphate

Intermediate **10.1a** has 3 chiral centers with a (2R),(6S),(10S)-configuration. The configuration of all three chiral centers originates from the enantiomerically pure starting materials (S)-(+)-1,2-isopropylidene-glycerol (sn-1,2-IPG), (S)-(-)-glycidol and (R)-(+)-glycidol and is transferred without isomerization in the final product DPPG₃. The (10S) configuration in is defined by the use sn-1,2-IPG. The use of the enantiomer, (R)-(-)-1,2-o-isopropylideneglycerol, would lead to (10R). The single stereoisomer is obtained in 7 steps with the following synthesis:

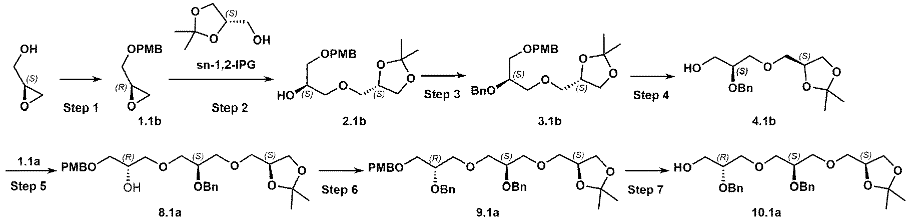

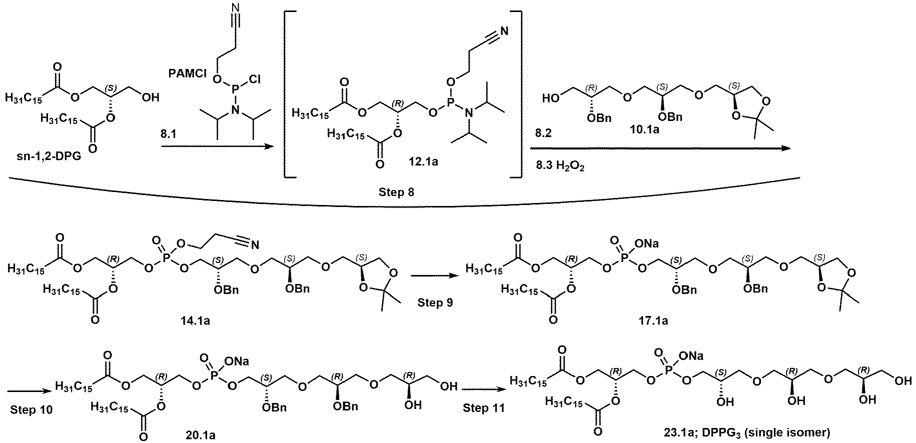

### Step 1:

(S)-(-)-glycidol is dissolved in NMP, then PMBCI is added followed by NaH at 0-5 °C. The resulting reaction mixture is stirred at RT until completion of reaction. Then the mixture is poured into ice water and extracted with EtOAc. The organic phase is washed with brine, dried over Na₂SO₄ and concentrated under reduced pressure. The isolated residue is purified by silica gel chromatography (n-Heptane/Ethyl acetate) to obtain **1.1b.**

The same procedure is used to obtain **1.1a** from (R)-(+)-glycidol.

### Step 2+3:

**1.1b** is dissolved in DMF, then *sn*-1,2-IPG is added, followed by tBuOK at 0-5 °C and the resulting reaction mixture is stirred at RT until completion of reaction to obtain **2.1b.** Subsequently, without work up or further purification additional tBuOK and benzyl bromide are added at 0-5 °C and the resulting reaction mixture stirred at RT until completion of reaction. Then, the reaction mixture is poured into ice water and extracted with EtOAc. The organic phase is washed with brine, dried over Na₂SO₄ and concentrated under reduced pressure. Pure compound **3.1b** is obtained after purification by silica gel chromatography (n-Heptane/Ethyl acetate).

### Step 4:

**3.1b** is dissolved in DCM/H₂O 10:1 (vol/vol) and the mixture cooled to 0 °C. 2,3-dichloro-5,6-dicyano-1,4-benzoquinone (DDQ) is added, and the mixture stirred at RT until completion of reaction. The reaction mixture is filtered, and the filtrate washed with aq. NaHCOs and water. The organic phase is dried over Na₂SO₄ and the solvent removed under reduced pressure. Crude **4.1b** is purified with silica gel chromatography (n-Heptane/Ethyl acetate).

### Step 5+6:

**1.1a** is dissolved in DMF, then **4.1b** is added, followed by tBuOK at 0-5 °C and the resulting reaction mixture s stirred at RT until completion of reaction to obtain **8.1a.** Subsequently, without work up or further purification additional tBuOK and benzyl bromide are added at 0-5 °C and the resulting reaction mixture stirred at RT until completion of reaction. Then, the reaction mixture is poured into ice water and extracted with EtOAc. The organic phase is washed with brine, dried over Na₂SO₄ and concentrated under reduced pressure. Pure compound **9.1a** is obtained after purification by silica gel chromatography (n-Heptane/Ethyl acetate).

### Step 7:

**9.1a** is dissolved in DCM/H₂O 10:1 (vol/vol) and the mixture cooled to 0 °C. 2,3-dichloro-5,6-dicyano-1,4-benzoquinone (DDQ) is added, and the mixture stirred at RT until completion of reaction. The reaction mixture is filtered, and the filtrate washed with aq. NaHCOs and water. The organic phase is dried over Na₂SO₄ and the solvent removed under reduced pressure. Crude **10.1a** is purified with silica gel chromatography (n-Heptane/Ethyl acetate).

### Step 8:

DIPEA is added to a suspension of sn-1,2-DPG in MeTHF and DCM, then then mixture is cooled to -15 °C. 2-Cyanoethyl-*N*,*N*-diisopropylchlorophosphoramidite (PAMCI) is dissolved in DCM and the solution cooled to -20 °C, before addition to the reaction mixture. The resulting mixture is stirred for 1 h at -15 °C, followed by 1.5 h at 0°C and 1 h at 20 °C. Then 4,5-dicyanoimidazole (DCI) and **10.1a** are dissolved in MeTHF and the solution slowly added at 20 °C to the reaction mixture. If necessary, an additional amount of DCI is added as solution in DCM after stirring for 1 h, then the whole mixture is stirred overnight at 20 °C. Then aqueous 35% w/w H₂O₂ solution is added at 0 °C over 1 h and then stirred at 20 °C for another 1 h. The reaction mixture is diluted with 5 wt% aq. Na₂S₂O₅ solution extracted with MeTHF. The organic phase is dried over Na₂SO₄ and the solvents are removed under reduced pressure. Crude intermediate **14.1a** is purified by flash silica chromatography.

### Step 9:

DBU is added to a solution of **14.1a** dissolved in MeTHF and the reaction mixture stirred at RT for 2 h. Then Pd/C is added, and the obtained reaction suspension is stirred under H₂ atmosphere (1.2 bar) for 1 h. The reaction mixture is filtered over celite and washed with 1M aq. citric acid solution and organic phase separated. The organic phase is washed with 5 wt%. aq. NaHCOs solution, dried over Na₂SO₄ and concentrated under reduced pressure to yield **17.1a.**

### Step 10:

Crude **17.1a** is suspended in 2-propanol, then TFA is added, and the obtained reaction mixture stirred at 45 °C for 1 h. The TFA is removed under reduced pressure and the crude product twice co-distilled with 2-propanol, then dissolved in MeTHF and the pH adjusted to 7 with 8wt% NaHCOs solution. The organic phase is separated, dried over Na₂SO₄ and concentrated under reduced pressure to obtain crude **20.1a.**

### Step 11:

**20.1a** is dissolved in MeTHF and then washed with 8 wt% NaHSO₄ solution, followed by adjustment of the pH to 4 with NaOAc in water. The organic phase is separated and concentrated. A suspension of 20% Pd(OH)₂ on charcoal (50% wet) in MeOH is added and the obtained suspension is stirred under H₂ atmosphere (3.5 bar) until completion of reaction. The pH is adjusted to pH 7 with addition of NaOAc and then the mixture is filtered over decalite and concentrated under reduced pressure. The concentrate is diluted with DCM and filtered over decalite again and then the organic phase is separated and concentrated under reduced pressure. Crude **DPPG₃** is purified by flash silica chromatography.

### 1.4 Stereospecific cleavage of DPPG₂ with Phospholipase A2

Phospholipases are enzymes that selectively cleave bonds in glycerophospholipids based on their stereochemistry. They play a role in the metabolization of glycerophospholipids in vivo. Phospholipase A2 hydrolyzes 1,2-acyl-*sn*-glycero-3-phospholipids to their 1-acyl-sn-glycero-3-phospholipid and the corresponding fatty acid. For the experiments, commercially available enzyme (extracted from porcine pancreas) was used. Lipid samples (2 µmol) were dissolved 1.5 wt% deoxycholate and subsequently incubated in Tris-buffer pH 8.5 supplemented with CaCl₂ together with 0.5 U enzyme in a thermoshaker at 37°C. At distinct time points, samples were taken and cleavage was analyzed by HPLC. A Waters XBridge Phenyl column (3.5 µm, 2.1*150 mm) was used and analyte detection was achieved with a charged aerosol detector (CAD). The analytes are separated using a gradient elution using 100 mM NH₄Ac pH 6.0 in water and methanol as eluent A and eluent B, respectively. Column oven was set to 35°C.

Three DPPG₂ batches differing in configurations of the stereogenic centers in their diglycerol headgroup were tested and DPPC was applied as control glycerophospholipid (Figure 2). DPPC was cleaved notably slower than DPPG₂. DPPG₂ batches showed a trend for a slower cleavage depending on the configuration of the stereogenic centers in the diglycerol headgroup. After 10 min, 14.8%, 20.4%, and 24.3% of DPPG₂ according to WO 9730058, WO 2014/202680 A1, and Formula III, respectively, was still detectable in the sample.

### 1.5 Manufacturing of DPPG₂-TSL-DOX

Thermosensitive liposomes (TSL) and compositions thereof, also called thermosensitive (TSL) formulations, can be prepared by distinct preparation processes (e.g., lipid film hydration and extrusion method, ethanol injection, or other methods). Active pharmaceutical ingredients are either loaded passively or by an active process, respectively. Active remote loading can be achieved by using, for example, an ammonium gradient, an acid gradient, or an EDTA salt gradient. Preparation methods for TSL with encapsulated DOX (DPPG₂-TSL-DOX) are described in WO 2022/008471 A1.

### 1.5.1 Lipid film hydration method with DPPG₂ according to WO 97/30058

DPPG₂ according to WO 97/30058 was used in the batch preparations. 15 batches were prepared using 100 nm filter membranes during extrusion. 25 batches were prepared using 200 nm filter membranes during extrusion. These batches were used for comparison purposes (Hossann et al. 2021).

All solutions were prepared with deionized and purified water from the ultrapure water system (Milli Q Advantage, Millipore) and subsequently filtered through 0.2 µm before usage. Phospholipids in the desired molar ratio (DPPC/DSPC/DPPG₂ 50:20:30) were dissolved in chloroform/methanol 9:1 (vol/vol) using a round-bottomed flask. The solvent was evaporated under vacuum in a rotary evaporator until a thin and homogeneous lipid film was formed. The lipid film was dried for at least 1 hour at 10 mbar/70°C to remove remaining traces of organic solvent. Hydration of the film was performed with 300 mM citric acid pH 4 at 60 °C for 30 min under shaking. The resulting lipid concentration was 50 mM. Unilamellar vesicles were obtained by 10 times extrusion at 60°C with maximum 20 bar N₂ pressure through two polycarbonate filters of desired pore size (e.g., 100 nm, 200 nm, Whatman, GE Healthcare Europe GmbH, Freiburg, Germany) using a thermobarrel extruder (LIPEX^{™}, Northern Lipids Inc. Burnaby, BC, Canada). Subsequently, the dispersion was cooled to 2-8°C and buffer was exchanged to physiological phosphate-buffered saline (PBS) buffer pH 7.4 applying PD10 columns (GE Healthcare). The desired active DOX loading conditions were obtained by mixing the liposomal dispersion with PBS pH 7.4 and the DOX stock solution (5.7 mg/ml DOX HCl). The dispersion was heated under shaking (e.g., 37°C up to 60 min) in an Eppendorf Thermomixer comfort with a 50 ml thermoblock. Traces of unencapsulated DOX were removed by centrifugation at 75,000 xg (Beckman Coulter Avanti J-26 XP with a JA-25.50 rotor). The supernatant was discarded, and the pellet was carefully resuspended with storage buffer (e.g., 10% (w/v) trehalose, 10.5 mM Na/K phosphate pH 7.4) in case storage at -20°C was intended. If batches were used immediately, buffer exchange was performed with PBS pH 7.4.

### 1.5.2 Ethanol injection method with using stereoisomeric DPPG₂

Stereoisomeric DPPG₂ according to Formula III was used in 8 independent batch preparations as follows.

DPPG₂-TSL-DOX have been prepared and characterized as described in WO 2022/008471 A1. All applied solutions were prepared with deionized and purified water from an ultrapure water system and subsequently filtered through 0.2 µm before usage. The pH of 300 mM ammonium phosphate buffer and physiological PBS buffer was adjusted to 7.4 with phosphoric acid if required, respectively. Phospholipid excipients in molar ratio DPPC/DSPC/stereoisomeric DPPG₂ 50:20:30 were dissolved in ethanol at 60°C. The ethanolic lipid solution and the ammonium phosphate buffer were independently pumped through two heating coils (60°C) and the streams were combined at 60°C with a T-joint. Subsequently, the 60°C hot dispersion was pumped through a 60°C hot extrusion chamber equipped with multiple extrusion membranes of appropriate pore size to generate liposomes with a vesicle size in the range of 100 to 150 nm. After extrusion, the stream was pumped through a 5°C cold cooling coil into an ice-cooled collector vessel. The gradient for active loading of DOX was generated by an extraliposomal buffer exchange to PBS pH 7.4 with tangential flow filtration (TFF). The desired molar DOX:lipid ratio of 0.08 was obtained by mixing the liposomal dispersion with PBS buffer pH 7.4 and the aqueous DOX stock solution (5.7 mg/ml DOX HCl). The dispersion was heated under stirring at 37°C for 30 min in a round bottom flask equipped with a heating mantle. After cooling of the dispersion to 2-8°C, traces of unencapsulated DOX were removed and extraliposomal buffer was exchanged to the storge buffer by TFF. Finally, the dispersion underwent aseptic filtration (0.2 µm).

### 1.5.3 Ethanol injection method with DPPG₂ according to WO 97/30058

Liposomal batches by using DPPC, DSPC and DPPG₂ (according to WO 97/30058) in a molar ratio of 50:20:30 were prepared as control. These liposomes were loaded with DOX as described in Example 1.5.2 using 250 mM ammonium sulfate pH 5.4 (4 batches) or 300 mM ammonium phosphate pH 7.4 (4 batches) as loading buffer. No buffer exchange to storage buffer was performed because the batches were immediately characterized and no storage at -20°C was intended.

### 1.6 In vitro characterization of TSL preparations

The TSL preparations produced in Examples 1.5.1, 1.5.2, and 1.5.3 have been characterized as follows. The hydrodynamic diameter (z average), size intensity distribution plot and zeta potential were determined by dynamic light scattering (DLS, Zetasizer Nano ZS, Malvern Instruments, Worcestershire, United Kingdom). Purity and concentration of DOX and the phospholipid excipients have been quantified with HPLC equipped with a fluorescence detector or a charged aerosol detector (CAD), respectively. In vitro temperature-dependent DOX release (TDR) was analyzed in fetal calf serum (FCS). Residual solvents were measured with gas chromatography (headspace). Additionally, pH, osmolarity, bacterial endotoxin, and sterility were measured with the corresponding European Pharmacopeia (EP) methods.

All batches were compared regarding their biophysical characteristics to evaluate the influence of stereochemistry and/or production method, respectively. The results are depicted in Figure 3.

Vesicle size (expressed as z average) was 118 ± 8 nm, 119 ± 2 nm, 120 ± 11 nm, and 135 ± 14 nm for batches prepared with ethanol injection (DPPG₂ according to formula III), ethanol injection (DPPG₂ according to WO 97/30058), lipid film hydration (100 nm extrusion), and lipid film hydration (200 nm extrusion) preparation method, respectively. The PDI was 0.10 ± 0.03, 0.13 ± 0.04, 0.13 ± 0.07, and 0.13 ± 0.05, respectively. The zeta potential was -28.3 ± 1.2 mV, -22.7 ± 7.0 mV, and -26.3 ± 3.5 mV for batches prepared with ethanol injection (DPPG₂ according to formula III), lipid film hydration (100 nm extrusion), and lipid film hydration (200 nm extrusion) preparation method, respectively.

Overall, the ethanol injection preparation method resulted in TSL formulations with smaller variation in vesicle size, size distribution, and zeta potential (low standard deviation) compared to the lipid film hydration preparation method. This indicates the superior robustness and controllability of the ethanol injection preparation process.

All prepared batches showed a temperature-dependent DOX release profile of TSL formulations (Figure 3). There are no notable differences in the TDR for batches produced by the lipid film hydration method but differing in their vesicle size (expressed as z average). Thus, DOX release kinetics of DPPG₂-based TSL were not affected by the vesicle size in the range of 117 nm to 161 nm (investigated temperature range: 37 to 41°C) (Hossann et al. 2010). When comparing batches from different preparation methods it is important to mention for data interpretation that the excipients used for DOX loading do not have an influence on the TDR (WO 2022/008471 A1).

*In vitro* DOX release during 5 min at 37°C in FCS was 6.8 ± 3.6 %, 1.1 ± 1.2 %, 3.5 ± 3.5 %, and 6.5 ± 6.2 % for batches prepared with ethanol injection (DPPG₂ according to formula III), ethanol injection (DPPG₂ according to WO 97/30058), lipid film hydration (100 nm extrusion), and lipid film hydration (200 nm extrusion) preparation methods, respectively. There was a minor effect on the stability at body temperature between the batches, but all showed acceptable low DOX release < 10%. *In vitro* DOX release during 5 min at 40°C in FCS was 75.4 ± 9.9 %, 65.7 ± 8.5 %, 46.7 ± 26.2 %, and 43.6 ± 23.3 %, respectively. All batches showed a comparable heat-induced DOX release at temperatures >40°C with more than 75% release during 5 min of incubation (data not shown).

Surprisingly, batches prepared with ethanol injection and using DPPG₂ according to Formula III showed higher *in vitro* DOX release at 37°C, 38°C, 39°C, and 40°C during 5 min in FCS compared to batches prepared with ethanol injection and using DPPG₂ according to WO 97/30058. This indicates that DOX is transferred faster across the membrane bilayer in batches produced with DPPG₂ according to Formula III than in batches produced with DPPG₂ according to WO 97/30058. This might be caused by a more homogenous packing of the lipid excipients in the bilayer, resulting in a narrower solid gel to liquid disordered phase transition when heating the TSL above their Tₘ. The faster drug release under hyperthermia is a favorable effect for the *in vivo* application of such TSL, since the encapsulated drug is faster bioavailable in the heated body area and more DOX can be accumulated in the tumor tissue.

### 1.7 Protein corona of DPPG₂-TSL preparation

The molar content of DPPG₂ influences the composition of the protein corona of DPPC/DSPC/DPPG₂ 80-x/20/x (mol/mol) (x = 5, 10, 20, 30). A potential stereoselective effect for the binding of proteins to DPPG₂-based TSL is investigated.

### 1.8 In vivo examination of DPPG₂-TSL-DOX in a rat model

DPPG₂-TSL-DOX batches produced by either the ethanol injection preparation or lipid film hydration method (comparison example) were investigated in the rat BN175 sarcoma model. The former batches contained DPPG₂ according to Formula III whereas the latter batches contained DPPG₂ according to WO 97/30059, respectively. Batches had been characterized as described in example 1.6. DPPG₂-TSL-DOX (DPPG₂ according to Formula III) prepared by the ethanol injection showed a vesicle size (z average) of 118 nm, a PDI of 0.08, a zeta potential of -28.3 mV, and a DOX:lipid ratio of 0.06, respectively. DPPG₂-TSL-DOX (DPPG₂ according to WO 97/30059) prepared by the lipid film hydration method showed a vesicle size (z average) of 178 nm, a PDI of 0.13, a zeta potential of -29.4 mV, and a DOX:lipid ratio of 0.10, respectively.

For the PK study, 18 anaesthetized healthy male Brown Norway rats (-300 g) received a dose of 2 mg/kg DOX of either non-liposomal DOX (n = 6), DPPG₂-TSL-DOX (DPPG₂ according to formula III) (n = 6), or DPPG₂-TSL-DOX (DPPG₂ according to WO 97/30059) (n = 6) as i.v. bolus injection, respectively. Body temperature of the animals was controlled via a rectal temperature probe. During anesthesia, the body temperature of animals was maintained by a water bath (37°C). Blood samples were taken at defined time points. DOX and DPPG₂ content were measured in plasma by HPLC equipped with a fluorescence detector and LC/MS, respectively.

DOX plasma clearance of both DPPG₂-TSL-DOX batches was similar (Figure A) and showed the expected long-circulating properties. Plasma half-life (t_{1/2,α}) of DOX was 141 ± 5 min and 180 ± 14 min for DPPG₂-TSL-DOX (DPPG₂ according to formula III) and DPPG₂-TSL-DOX (DPPG₂ according to WO 97/30059), respectively. After 240 min, the plasma concentration of the percentage of the initial dose (%ID) was also similar with 6.3% and 17.2%, respectively, indicating a comparable clearance of DOX with both formulations. The slight difference in t_{1/2,α} can be explained by the known effect of vesicle size on the circulation half-life of drugs encapsulated in DPPG₂-TSL (Limmer et al. 2014). In contrast, non-liposomal DOX was cleared within minutes with no DOX plasma levels detectable at time points >15 min.

The difference in molar DOX:lipid ratio in both DPPG₂-TSL-DOX formulations resulted in a different maximum observed concentration (Cₘₐₓ) for DPPG₂ (Figure B). The administered amount of DPPG₂ was higher for DPPG₂-TSL-DOX (DPPG₂ according to formula III). This was because of the smaller DOX:lipid ratio in this formulation and that all rats had been dosed with the same amount of DOX (2 mg/kg). Both liposomal formulations demonstrated a similar DPPG₂ clearance with a comparable plasma concentration after 240 min.

High blood levels of DPPG₂ (nanocarrier) and DOX (drug) in the first hour after intravenous (i.v.) application is an important prerequisite for TSL formulations (Kneidl et al. 2014). This would allow activation of as many of the dosed TSL by local or regional HT in the tumor and subsequently generate high local DOX levels. Only DOX that is released in the tumor will be therapeutically effective (see below).

In an independent set of PK experiments, 12 anaesthetized healthy male Brown Norway rats (-300 g) received DPPG₂-TSL-DOX (DPPG₂ according to formula III) with a dosage of 2 mg/kg DOX i.v. as bolus injection. Six rats received a regional HT treatment (41.5°C, water bath) of one hind leg, whereas the other 6 rats received a regional NT treatment (37°C, water bath) of one hind leg, respectively. The body temperature of these animals was controlled via a rectal temperature probe. Blood samples were taken at defined time points. DOX content was measured in plasma by HPLC equipped with a fluorescence detector. It was demonstrated that the 1-hour regional HT treatment was sufficient to deplete circulating DPPG₂-TSL-DOX by heat-induced DOX release to almost completeness. PK parameters dropped notably, indicating a notable heat-induced DOX release from the nanocarriers in the heated (41.5°C) hind leg of the rats. For example, t_{1/2,α} decreased from 141 ± 5 min to 29 ± 3 min for rats receiving NT and HT, respectively. The area under the concentration curve (AUC) in the first 60 min decreased from 1410 to 412 µg*h/ml, respectively. The induced DOX release in the heated body part was demonstrated for DPPG₂-TSL-DOX (DPPG₂ according to WO 97/30059) before (Hossann et al. 2021).

The accumulation of DOX in the tumor tissue (Figure D) and selected organs (Figure E) were investigated next. Eighteen anaesthetized tumor-bearing male Brown Norway rats (-300 g) received a dose of 2 mg/kg DOX of either non-liposomal DOX (n = 6), DPPG₂-TSL-DOX (DPPG₂ according to formula III) (n = 6), or DPPG₂-TSL-DOX (DPPG₂ according to WO 97/30059) (n = 6) as i.v. bolus injection, respectively. All animals had two BN175 tumors (soft tissue sarcoma) subcutaneously implanted to each hind leg, respectively. One tumor received 60 min of a local HT treatment with a light source of 41.5°C, whereas the second tumor remained untreated (room temperature, NT). The light source was used instead of a water bath to apply a HT treatment, because it allows a more focused (local) heating of a tumor tissue (Willerding et al. 2016). Animals were sacrificed after 60 min, organs were perfused, and tumors and organs were collected. DOX content was measured in plasma by HPLC equipped with a fluorescence detector.

The DOX concentration in the tumor strongly depended on the applied condition (HT vs. NT) and formulation (non-liposomal vs. TSL). HT application increased DOX tumor accumulation (compared to the content in the untreated tumors) 1.6-fold, 16.2-fold, and 12.7-fold for rats treated with non-liposomal DOX, DPPG₂-TSL-DOX (DPPG₂ according to WO 97/30059), and DPPG₂-TSL-DOX (DPPG₂ according to formula III), respectively. Enrichment factors were comparable with published data (Willerding et al. 2016, Hossann et al. 2021). DOX concentration in the heated tumors was 3.1 ± 0.5 ng/mg, 32.3 ± 23.0 ng/ml, and 41.7 ± 19.0 ng/ml, respectively. DOX concentration in the untreated tumors was 1.9 ± 0.3 ng/mg, 2.0 ± 0.3 ng/ml, and 3.3 ± 0.3 ng/ml, respectively. Interestingly, the DOX content in both tumors in rats treated with DPPG₂-TSL-DOX (DPPG₂ according to formula III) was higher compared to the corresponding tumors in rats treated with DPPG₂-TSL-DOX (DPPG₂ according to WO 97/30059). This fits nicely with the *in vitro* DOX release data (example 1.6) that indicated that DOX can be made bioavailable faster for DPPG₂-TSL-DOX (DPPG₂ according to formula III).

The DOX organ distribution was comparable for both DPPG₂-TSL-DOX formulations, except for the spleen. The DOX content in the spleen was significantly lower for the DPPG₂-TSL-DOX (DPPG₂ according to formula III) with 13.7 ± 1.0 ng/mg compared to 20.6 ± 2.4 ng/mg for the DPPG₂-TSL-DOX (DPPG₂ according to WO 97/30059). It was previously demonstrated that the preparation method had no influence on the organ distribution (including the spleen content) (WO 2022/008471 A1), indicating that usage of DPPG₂ according to Formula III might reduce the uptake rate of the liposomes into the spleen.

In literature, there are examples that even minor changes in the molecular structure can yield to unexpected strong effects *in vitro* or *in vivo.* Replacing DPPG₂ with DPPG₄ in DPPC/DPPGₓ/cholesterol 40:10:50 (mol/mol) liposomes increases uptake into the spleen of rats (Schagon 1997).

Application of non-liposomal DOX resulted in significantly higher DOX accumulation in lung and heart compared to both DPPG₂-TSL-DOX formulations (Figure 4E). This might lower the risk for DOX-related cardiotoxicity after application of DPPG₂-TSL-DOX.

Finally, the therapeutic efficacy of both DPPG₂-TSL-DOX formulations was investigated *in vivo* and compared to selected clinically relevant DOX formulations (e.g., non-liposomal DOX, Caelyx). 36 anaesthetized tumor-bearing male Brown Norway rats were treated with DPPG₂-TSL-DOX (DPPG₂ according to formula III) with HT (n = 6), DPPG₂-TSL-DOX (DPPG₂ according to WO 97/30059) with HT (n = 6), non-liposomal DOX with HT (n = 6), Caelyx with HT (n=6), physiological saline with HT (n = 6), and DPPG₂-TSL-DOX (DPPG₂ according to formula III) without HT (n = 6), respectively. All animals had one BN175 tumor (soft tissue sarcoma) s.c. implanted to one hind leg. Local HT treatment was performed with a light source. Target temperature was 41 °C. Animals were preheated to this temperature for 30 min and temperature was maintained at 41 °C for 60 min after i.v. bolus injection. Animals treated with a DOX formulation received 2 mg/kg DOX as single i.v. bolus injection after one tumor diameter was > 5 mm. After the end of the treatment, animals were placed back into a cage and tumor growth was monitored every 2^{nd} day until reaching an end point (e.g., tumor diameter > 3 cm, exulzeration, bleeding, skin toxicity).

Figure 4F depicts the Kaplan-Meier plot (survival, 5-fold increase in tumor volume) of all experimental groups. DPPG₂-TSL-DOX (DPPG₂ according to formula III) in combination with HT reached longest survival, followed by DPPG₂-TSL-DOX (DPPG₂ according to WO 97/30059) in combination with HT treatment. This might be due to the faster DOX release at 40°C and the reduced spleen uptake compared to the DPPG₂-TSL-DOX (DPPG₂ according to WO 97/30059). In absence of HT, therapeutic efficacy of DPPG₂-TSL-DOX (DPPG₂ according to formula III) was notably reduced and comparable to 0.9% saline, Caelyx, and non-liposomal DOX, respectively. The only rats that showed a transient tumor size reduction after treatment had been applied with DPPG₂-TSL-DOX formulations when combined with HT. All other rats showed a steady tumor volume increase after the treatment. These results indicate the necessity of high local DOX concentrations in the tumor tissue for an efficient tumor treatment.

In conclusion, DPPG₂-TSL-DOX (DPPG₂ according to formula III) showed a slightly faster DOX clearance from the blood stream compared to DPPG₂-TSL-DOX (DPPG₂ according to WO 97/30059). Surprisingly, the DOX accumulation into the tumor tissue was increased, the DOX content in the spleen reduced, and the formulation showed its superiority in therapeutic efficacy.

The present application also discloses the following items:
Item 1. A stereoisomeric phosphatidyloligoglycerol of Formula (I) 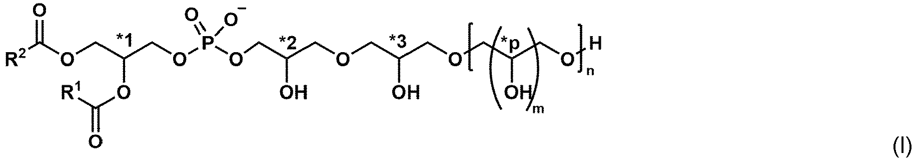
   wherein the three stereogenic centers *1, *2 and *3 have a predetermined configuration; and *p denotes further stereogenic centers; and
   wherein R¹ and R² each independently are a hydrocarbon group having 12 to 24 C-atoms; n is a number from 0 to 20; and
   m at each occurrence independently is 0 or 1.
Item 2. The stereoisomeric phosphatidyloligoglycerol according to Item 1 having Formula (I), wherein n is a number from 0 to 10, preferably wherein n is a number from 0 to 5, more preferably wherein n is 0, 1 or 2, even more preferably wherein n is 0 or 1.
Item 3. The stereoisomeric phosphatidyloligoglycerol according to any one of Items 1 or 2 having Formula (I), wherein n is 0.
Item 4. The stereoisomeric phosphatidyloligoglycerol according to any one of Items 1 - 3 Formula (I), wherein m at each occurrence is 1.
Item 5. The stereoisomeric phosphatidyloligoglycerol according to any one of Items 1 - 4 having Formula (I), wherein R¹ and R² each independently are a hydrocarbon group having 13 to 19 C-atoms, in particular 14 to 18 C-atoms.
Item 6. The stereoisomeric phosphatidyloligoglycerol according to any one of Items 1 - 5 having Formula (I), wherein R¹ and R² each independently are a linear or branched hydrocarbon group, preferably a linear hydrocarbon group and/or wherein R¹ and R² each independently are a linear or branched alkyl group, in particular a linear alkyl group.
Item 7. The stereoisomeric phosphatidyloligoglycerol according to any one of Items 1 - 6 having Formula (I), wherein R¹ and R² are a saturated or a monounsaturated or a polyunsaturated alkyl group, preferably a saturated alkyl group.
Item 8. The stereoisomeric phosphatidyloligoglycerol according to any one of Items 1 - 7 having Formula (I), wherein R¹ and R² each independently are a linear saturated alkyl group; preferably, R¹ and R² each independently are a linear saturated C12 to C24 alkyl group; more preferably R¹ and R² are a linear saturated C13 to C19 alkyl group; and most preferably, R¹ and R² are a linear saturated C15 alkyl group.
Item 9. The stereoisomeric phosphatidyloligoglycerol according to any one of Items 1 - 8 having Formula (I), wherein stereogenic center *1 is in R-configuration, stereogenic center *2 is in S-configuration and stereogenic center *3 is in R-configuration.
Item 10. The stereoisomeric phosphatidyloligoglycerol according to any one of Items 1 - 9 having Formula (I), wherein the stereoisomeric phosphatidyloligoglycerol is a single stereoisomer.
Item 11. The stereoisomeric phosphatidyloligoglycerol according to any one of Items 1-10 having Formula (I), wherein the stereogenic centers *p have a predetermined configuration.
Item 12. The stereoisomeric phosphatidyloligoglycerol according to any one of Items 1-11 having Formula (II) 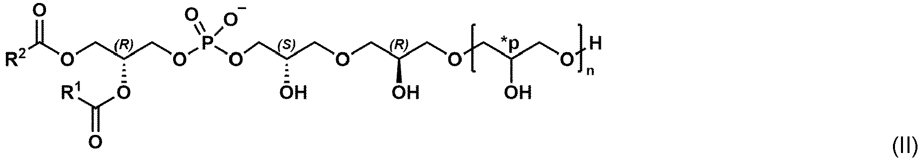 wherein stereogenic center *1 is in R-configuration, stereogenic center *2 is in S-configuration and stereogenic center *3 is in R-configuration and *p denotes further stereogenic centers.
Item 13. The stereoisomeric phosphatidyloligoglycerol according any one of Item 1 -12, wherein n = 0 or 1, and in particular, wherein n = 0.
Item 14. The stereoisomeric phosphatidyloligoglycerol according to any one of Items 1-13, wherein R¹ and R² independently are a linear saturated alkyl group having 13 to 19 C-atoms and, in particular, wherein R¹ and R² are a linear saturated C15-alkyl group.
Item 15. The stereoisomeric phosphatidyloligoglycerol according to any one of Items 1-14 having Formula (III) 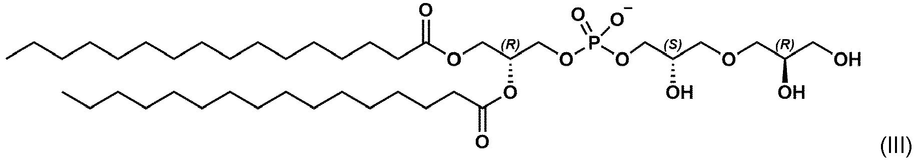
Item 16. The stereoisomeric phosphatidyloligoglycerol according to any one of Items 1-14 having Formula (III') 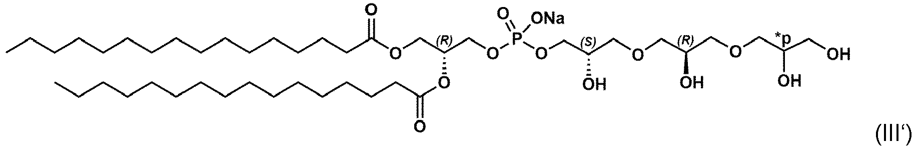 wherein the stereogenic center *p has a predetermined configuration.
Item 17. A method for preparing a stereoisomeric phosphatidyloligoglycerol according to any one of Items 1-16, characterized in that the synthesis route includes a central intermediate of Formula (IV) or of Formula (IV') 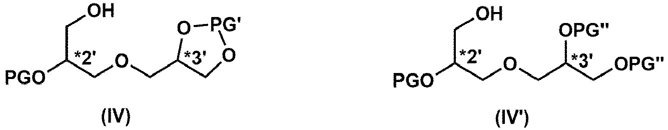 wherein the two stereogenic centers *2' and *3' have a predetermined configuration and wherein PG, PG' and PG" independently denote a protecting group.
Item 18. A method for preparing a stereoisomeric phosphatidyloligoglycerol according to Items 17, wherein PG at each occurrence independently is benzyl (Bn), tetrahydropyranyl (THP), ethoxyethyl (EE), 2-methoxypropan-2-yl (MOP), silyl or para-methoxybenzyl (PMB), preferably PG is benzyl (Bn); wherein PG" at each occurrence independently is benzyl (Bn), tetrahydropyranyl (THP), ethoxyethyl (EE), 2-methoxypropan-2-yl (MOP), silyl or para-methoxybenzyl (PMB), preferably PG" is benzyl (Bn); and wherein PG' is CH₂ (methylene), CHCH₃ (ethylidene), CHPh (benzylidene), CHPhp-Ome (para-methoxybenzylidene), C(CH₃)₂ (isopropylidene) or Si*t*Bu₂ (di-tert-butylsilylene), Si(iPr)₂-O-Si(iPr)₂ (tetraisopropyldisiloxanylidene), preferably PG' is C(CH₃)₂.
Item 19. The method for preparing a stereoisomeric phosphatidyloligoglycerol according to Item 17 or 18, characterized in that the synthesis route includes a central intermediate of Formula (lVa) or of Formula (IVa') 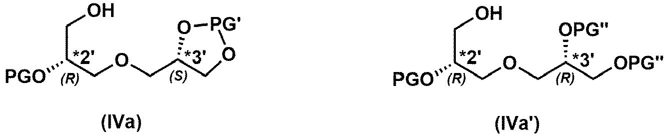 wherein stereogenic center *2' is in R-configuration and stereogenic center *3' is in S-configuration for (IVa) and in R-configuration for (IVa') and wherein PG, PG' and PG" independently denote a protecting group.
Item 20. The method for preparing a stereoisomeric phosphatidyloligoglycerol according to any one of Items 17 - 19, characterized in that the intermediate of Formula (IV) is prepared from glycidol and 1,2-isopropylidene-glycerol, in particular from (R)-(+)-glycidol and (S)-(+)-1,2-isopropylidene-glycerol (*sn*-1,2-IPG).
Item 21. The method for preparing a stereoisomeric phosphatidyloligoglycerol according to any one of Items 17-20, comprising reacting the central intermediate of Formula (IV) or (IVa) with a compound of Formula (V) 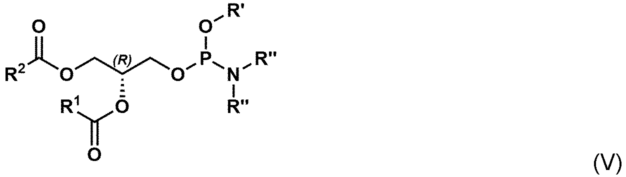 wherein R¹ and R² each independently are a hydrocarbon group having 12 to 24 C-atoms, R' is a leaving group and R" is a hydrocarbon group having 1 to 12 C-atoms.
Item 22. The method for preparing a stereoisomeric phosphatidyloligoglycerol according to any one of Items 17-21, wherein R' is 2-cyanoethyl (CNE) and R" is a C1-C12 hydrocarbon group, in particular R" is methyl (Me), ethyl (Et), isopropyl (iPr) or benzyl (Bn), preferably R" is isopropyl.
Item 23. A liposome comprising a stereoisomeric phosphatidyloligoglycerol according to any one of Items 1-16.
Item 24. The liposome comprising a stereoisomeric phosphatidyloligoglycerol according to Item 23, wherein the stereoisomeric phosphatidyloligoglycerol is a single stereoisomer of Formula (I).
Item 25. The liposome according to Items 23 or 24, wherein the stereoisomeric phosphatidyloligoglycerol is a single stereoisomer of Formula (III) 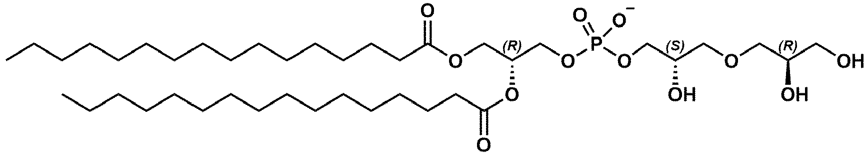
Item 26. The liposome according to any one of Items 23 - 25, further comprising at least one phosphatidylcholine of Formula (VII) 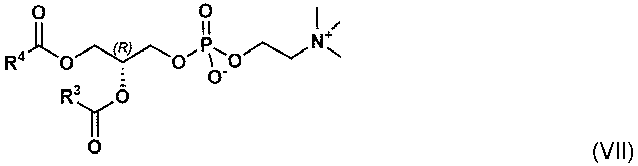 wherein R³ and R⁴ each independently are a hydrocarbon group having 12 to 24 C-atoms.
Item 27. The liposome according to any one of Items 23 - 26 comprising
   from 20 to 40 mol% of a stereoisomeric phosphatidyloligoglycerol according to any one of claims 1-16, in particular of phosphatidyloligoglycerol of Formula (III) being a single stereoisomer;
   from 40 to 60 mol% 1.2-dipalmittoylphosphatidylcholine; and
   from 15 to 25 mol% 1.2-distearoylphosphatidylcholine,
   based on the lipid content of the liposomes.
Item 28. The liposome according to any one of Items 23 - 26 comprising
   from 25 to 35 mol % of stereoisomeric DPPG₂ having Formula (III); and
   from 65 to 75 mol % of 1,2-dipalmitoyl-*sn*-glycero-3-phosphocholine (DPPC),
   based on the lipid content of the liposomes.
Item 29. The liposome according to any one of Items 23 - 28, being a thermosensitive liposome.
Item 30. The liposome according to any of Items 23 - 29 further comprising an active agent, in particular doxorubicin.

## Claims

1. A stereoisomeric phosphatidyloligoglycerol of Formula (I) 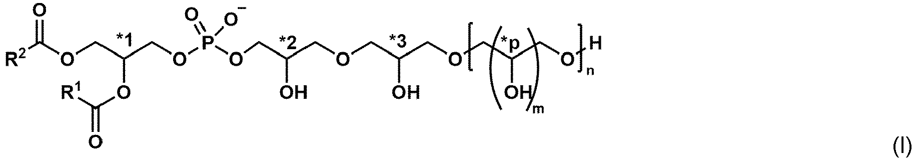
wherein the three stereogenic centers *1, *2 and *3 have a predetermined configuration; and *p denotes further stereogenic centers; and
wherein R¹ and R² each independently are a hydrocarbon group having 12 to 24 C-atoms; n is a number from 0 to 20; and
m at each occurrence independently is 0 or 1.

2. The stereoisomeric phosphatidyloligoglycerol according to claim 1 having Formula (II) 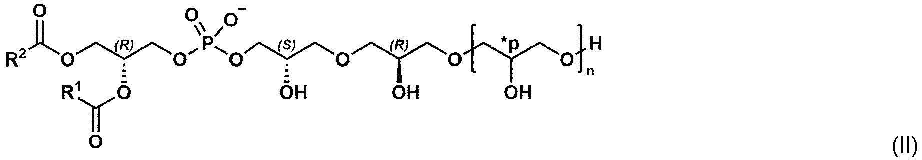 wherein stereogenic center *1 is in R-configuration, stereogenic center *2 is in S-configuration and stereogenic center *3 is in R-configuration and *p denotes further stereogenic centers.

3. The stereoisomeric phosphatidyloligoglycerol according to claim 1 or claim 2, wherein n = 0 or 1, and in particular, wherein n = 0.

4. The stereoisomeric phosphatidyloligoglycerol according to any one of claims 1-3, wherein R¹ and R² independently are a linear saturated alkyl group having 13 to 19 C-atoms and, in particular, wherein R¹ and R² are a linear saturated C15-alkyl group.

5. The stereoisomeric phosphatidyloligoglycerol according to any one of claims 1-4 having Formula (III)

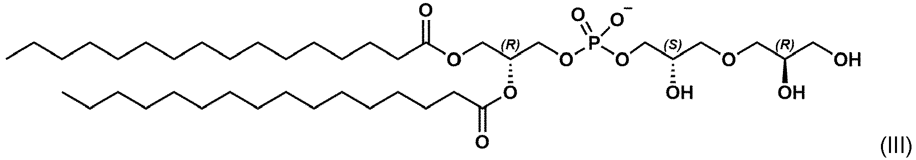

6. A method for preparing a stereoisomeric phosphatidyloligoglycerol according to any one of claims 1-5, **characterized in that** the synthesis route includes a central intermediate of Formula (IV) or of Formula (IV') 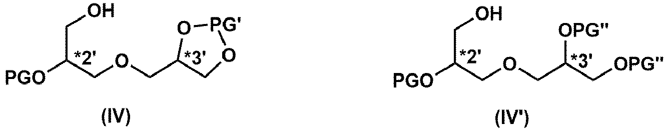 wherein the two stereogenic centers *2' and *3' have a predetermined configuration and wherein PG, PG' and PG" independently denote a protecting group.

7. The method for preparing a stereoisomeric phosphatidyloligoglycerol according to claim 6, **characterized in that** the synthesis route includes a central intermediate of Formula (IVa) or of Formula (IVa') 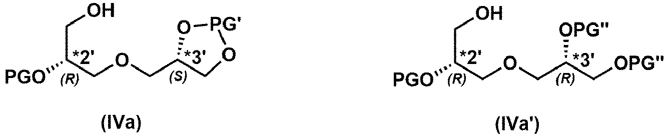 wherein stereogenic center *2' is in R-configuration and stereogenic center *3' is in S-configuration for (IVa) and in R-configuration for (IVa') and wherein PG, PG' and PG" independently denote a protecting group.

8. The method for preparing a stereoisomeric phosphatidyloligoglycerol according to claim 6 or 7, **characterized in that** the intermediate of Formula (IV) is prepared from glycidol and 1,2-isopropylidene-glycerol, in particular from (R)-(+)-glycidol and (S)-(+)-1,2-isopropylidene-glycerol (sn-1,2-IPG).

9. The method for preparing a stereoisomeric phosphatidyloligoglycerol according to any one of claims 6-8, comprising reacting the central intermediate of Formula (IV) or (IVa) with a compound of Formula (V) 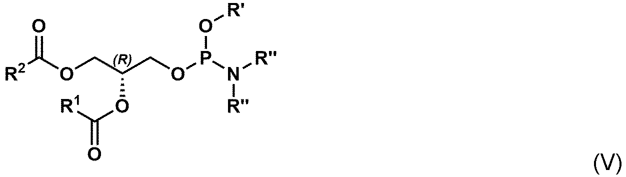 wherein R¹ and R² each independently are a hydrocarbon group having 12 to 24 C-atoms, R' is a leaving group and R" is a hydrocarbon group having 1 to 12 C-atoms.

10. A liposome comprising a stereoisomeric phosphatidyloligoglycerol according to any one of claims 1-5.

11. The liposome according to claim 10, further comprising at least one phosphatidylcholine of Formula (VII) 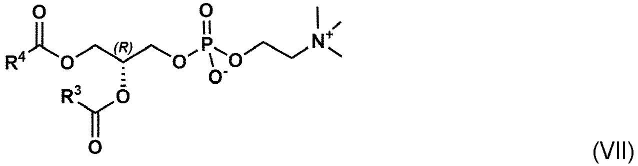 wherein R³ and R⁴ each independently are a hydrocarbon group having 12 to 24 C-atoms.

12. The liposome according to claim 10 or 11 comprising
20 to 40 mol% of a stereoisomeric phosphatidyloligoglycerol according to any one of claims 1-5, in particular of a stereoisomeric phosphatidyloligoglycerol of Formula (III);
40 to 60 mol% 1.2-dipalmitoylphosphatidylcholine; and
15 to 25 mol% 1.2-distearoylphosphatidylcholine.

13. The liposome according to any of claims 10-12 further comprising an active agent, in particular doxorubicin.
